(19) Europäisches Patentamt
European Patent Office
Office européen des brevets

(11) **EP 2 953 720 B1**

(12) **EUROPÄISCHE PATENTSCHRIFT**

(45) Veröffentlichungstag und Bekanntmachung des Hinweises auf die Patenterteilung:
**10.03.2021  Patentblatt 2021/10**

(21) Anmeldenummer: **14701743.8**

(22) Anmeldetag: **28.01.2014**

(51) Int Cl.:
**B01J 37/04** *(2006.01)*    **B01J 23/28** *(2006.01)*
**B01J 23/30** *(2006.01)*    **B01J 23/888** *(2006.01)*
**B01J 23/00** *(2006.01)*    **C07C 51/25** *(2006.01)*
**B01J 37/08** *(2006.01)*    **B01J 37/02** *(2006.01)*
**B01J 37/00** *(2006.01)*    **B01J 35/00** *(2006.01)*
**B01J 35/10** *(2006.01)*    **B01J 35/02** *(2006.01)*

(86) Internationale Anmeldenummer:
**PCT/EP2014/051556**

(87) Internationale Veröffentlichungsnummer:
**WO 2014/122043 (14.08.2014 Gazette 2014/33)**

(54) **VERFAHREN ZUR HERSTELLUNG EINER KATALYTISCH AKTIVEN MASSE, DIE EIN GEMISCH AUS EINEM DIE ELEMENTE MO UND V ENTHALTENDEN MULTIELEMENTOXID UND WENIGSTENS EINEM OXID DES MOLYBDÄNS IST**

METHOD FOR PRODUCING A CATALYTICALLY ACTIVE MASS THAT IS A MIXTURE OF A MULTI-ELEMENT OXIDE CONTAINING THE ELEMENTS MO AND V AND AT LEAST ONE OXIDE OF MOLYBDENUM

PROCÉDÉ DE FABRICATION D'UNE MASSE À ACTIVITÉ CATALYTIQUE QUI CONSTITUE UN MÉLANGE ENTRE UN OXYDE DE PLUSIEURS ÉLÉMENTS, LEQUEL CONTIENT LES ÉLÉMENTS MO ET V, ET AU MOINS UN OXYDE DU MOLYBDÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.02.2013   DE 102013202048**
**07.02.2013   US 201361761812 P**

(43) Veröffentlichungstag der Anmeldung:
**16.12.2015   Patentblatt 2015/51**

(73) Patentinhaber: **BASF SE**
**67056 Ludwigshafen am Rhein (DE)**

(72) Erfinder:
• **WELKER-NIEUWOUDT, Cathrin Alexandra**
**67134 Birkenheide (DE)**
• **DOBNER, Cornelia Katharina**
**67071 Ludwigshafen (DE)**

• **WALSDORFF, Christian**
**67061 Ludwigshafen (DE)**
• **MÜLLER-ENGEL, Klaus Joachim**
**76297 Stutensee (DE)**
• **MACHT, Josef**
**68159 Mannheim (DE)**

(74) Vertreter: **BASF IP Association**
**BASF SE**
**GBI-C006**
**67056 Ludwigshafen (DE)**

(56) Entgegenhaltungen:
**WO-A1-02/24327        WO-A1-2008/104577**
**WO-A2-2009/048553**

• **DATABASE WPI Week 201050 Thomson Scientific, London, GB; AN 2010-J16885 XP002723290, & JP 2010 155197 A (SUMITOMO CHEM CO LTD) 15. Juli 2010 (2010-07-15)**

EP 2 953 720 B1

**Beschreibung**

Die vorliegende Erfindung betrifft ein Verfahren zur Herstellung einer katalytisch aktiven Masse, die ein Gemisch aus einem die Elemente Mo und V enthaltenden Multielementoxid und weniastens einem Oxid des Molybdäns wie in den Ansprüchen definiert.

Mo und V enthaltende Multielementoxide sind im Stand der Technik bekannt (vgl. z.B. WO 2011 /134932 A1, DE 102012207811 A1, WO 2004/108267 A1, WO 2004/108284 A1, EP 714700 A2, DE 102005010645 A1, WO 95/11081 A1, DE 10350822 A1, US 2006/0205978 A1 und DE 102004025445 A1).

Sie eignen sich insbesondere als katalytisch wirksame Aktivmassen für die Katalyse der heterogen katalysierten partiellen Gasphasenoxidation von Acrolein zu Acrylsäure.

Die Bezeichnung Multielementoxid bringt dabei zum Ausdruck, dass die katalytisch wirksame Aktivmasse neben Mo, V und O (Sauerstoff) noch wenigstens ein weiteres chemisches Element enthält.

Der molare Anteil des Elements Mo an der Gesamtmenge aller von Sauerstoff verschiedenen Elemente des katalytisch wirksamen (aktiven) Multielementoxids beträgt dabei in der Regel 5 bis 95 mol-%, häufig 10 bis 90 mol-% und vielfach 15 bis 85 mol-% bzw. 20 bis 80 mol-%. Das molare Verhältnis von in einem solchen katalytisch aktiven Multielementoxid enthaltenem Mo zu im selben katalytisch aktiven Multielementoxid enthaltenem V (enthaltene molare Menge an Mo/enthaltene molare Menge an V) beträgt üblicherweise 15 : 1 bis 1 : 1, häufig 12 : 1 bis 2 : 1.

Aus dem Stand der Technik ist ferner bekannt, dass ein durch ein die Elemente Mo und V enthaltendes Multielementoxid katalysiertes Verfahren der heterogen katalysierten Gasphasenpartialoxidation von Acrolein zu Acrylsäure an ein und demselben Multielementoxid im Wesentlichen kontinuierlich über längere Zeiträume betrieben werden kann (vgl. z.B. DE 10350822 A1 und DE 102004025445 A1).

Allerdings verliert das Multielementoxid dabei mit zunehmender Betriebsdauer an katalytischer Wirksamkeit. Im Besonderen verschlechtert sich seine Aktivität.

Um das Partialoxidationsverfahren trotzdem möglichst lange an ein und derselben Aktivmasse betreiben zu können, wird im Stand der Technik auf unterschiedlichste Art und Weise versucht, diesem Alterungsprozess entgegenzuwirken.

Die EP 990636 A1 (z.B. Seite 8, Zeilen 13 bis 15) und die EP 1106598 A2 (z.B. Seite 13, Zeilen 43 bis 46) schlagen vor, die Minderung der Aktivität der Aktivmasse dadurch weitgehend auszugleichen, dass im Verlauf der Betriebszeit, unter ansonsten weitgehend gleichbleibenden Betriebsbedingungen, nach und nach die Reaktionstemperatur erhöht wird, um den Acroleinumsatz bei einmaligem Durchgang des Reaktionsgasgemischs durch das Katalysatorbett im Wesentlichen beizubehalten.

Nachteilig an der in der EP 990636 A1 sowie in der EP 1106598 A2 empfohlenen Verfahrensweise ist, dass sich mit zunehmender Erhöhung der Reaktionstemperatur der Alterungsprozess der Aktivmasse zunehmend beschleunigt (bestimmte Bewegungsvorgänge innerhalb der Aktivmasse, die zur Alterung beitragen, laufen z.B. zunehmend schneller ab).

Bei Erreichen eines Höchstwertes der Reaktionstemperatur muss der erschöpfte Multielementoxidkatalysator schließlich ausgetauscht werden. Nachteilig an einem solchen Austausch ist jedoch, dass er vergleichsweise aufwendig ist. Das Verfahren der Acrylsäureherstellung muss für längere Zeit unterbrochen werden und die Kosten der Multielementoxidherstellung sind ebenfalls erheblich.

Erwünscht sind daher Verfahrensweisen, die dabei behilflich sind, die Standzeit der Aktivmasse im Reaktor möglichst weitreichend zu verlängern.

Die DE 102004025445 A1 schlägt als ein Verfahren zum Langzeitbetrieb der heterogen katalysierten partiellen Gasphasenoxidation von Acrolein zu Acrylsäure vor, der Deaktivierung des Multielementoxids dadurch entgegen zu wirken, dass mit zunehmender Betriebsdauer der Aktivmasse der Arbeitsdruck in der Gasphase zunehmend erhöht wird. Nachteilig an dieser Verfahrensweise ist, dass mit zunehmendem Arbeitsdruck bei der heterogen katalysierten partiellen Gasphasenoxidation erhöhte Kompressorleistungen erforderlich sind.

Es wurde auch schon vorgeschlagen, mit zunehmender Betriebsdauer sowohl die Reaktionstemperatur als auch den Arbeitsdruck zu erhöhen.

Die EP 614872 A1 empfiehlt, die Standzeit des Multielementoxids dadurch zu verlängern, dass man nach mehrjähriger Betriebsdauer der Aktivmasse, mit der zum Zweck der Beibehaltung des Acroleinumsatzes (bezogen auf einen einmaligen Reaktordurchgang des Reaktionsgasgemischs) Erhöhungen der Reaktionstemperatur von 15 °C bis 30 °C und mehr einhergehen, das Verfahren der Partialoxidation unterbricht, und bei erhöhter Temperatur ein Regeneriergasgemisch aus Sauerstoff, Wasserdampf und Inertgas über und durch die Aktivmasse führt und anschließend die Partialoxidation bei verminderter Reaktionstemperatur fortsetzt (in diesem Zusammenhang sollen in dieser Schrift als Inertgase in einem Gasgemisch, das unter bestimmten Bedingungen durch ein Katalysatorbett geführt wird, ganz generell solche Gase verstanden werden, die bei der Hindurchführung des Gasgemischs durch das Katalysatorbett zu wenigstens 95 mol-%, bevorzugt zu wenigstens 98 mol-%, ganz besonders bevorzugt zu wenigstens 99 mol-% oder zu wenigstens 99,5 mol-%, oder zu 100 mol-% unverändert erhalten bleiben).

Nachteilig an der Verfahrensweise der EP 614872 A1 ist jedoch, dass bis zum Zeitpunkt der Verfahrensunterbrechung die Alterung des Multielementoxids ungebremst fortschreitet und gefördert wird. Außerdem bleibt der irreversible Alterungsanteil der Aktivmasse bei der Regenerierung unverändert erhalten.

Die DE 10350822 A1 versucht den Nachteilen der EP 614872 A1 dadurch wenigstens teilweise abzuhelfen, dass zum Ausgleich der Deaktivierung des Multielementoxids zwar auch jeweils die Reaktionstemperatur über die Betriebszeit erhöht wird, die Partialoxidation aber bereits jeweils unterbrochen wird, bevor diese Temperaturerhöhung 8 °C beträgt, um in regenerierender Weise ein molekularen Sauerstoff enthaltendes Gas über und durch das Multielementoxid zu führen. Nachteilig an der Verfahrensweise der DE 10350822 A1 ist jedoch, dass jede Regenerierung eine Unterbrechung des eigentlichen Partialoxidationsverfahrens erforderlich macht und irreversible Alterungsanteile ebenfalls nicht zu heilen vermag.

Nachteilig an allen vorstehend gewürdigten Verfahren des Standes der Technik zur Verlängerung der Standzeit eines zur Durchführung einer heterogen katalysierten Partialoxidation von Acrolein zu Acrylsäure als katalytisch wirksame Aktivmasse eingesetzten, die Elemente Mo und V enthaltenden, Multielementoxids ist zusätzlich, dass allesamt nicht versuchen, dem Eintritt einer Deaktivierung des Multielementoxids präventiv entgegenzuwirken, sondern erst dann einsetzen, wenn eine solche Deaktivierung des Multielementoxids bereits eingetreten ist, um der negativen Folgewirkung einer solchen Deaktivierung entgegenzuwirken.

Die WO 2008/104577 A1 offenbart dem gegenüber ein Verfahren, das der Deaktivierung eines die Elemente Mo und V enthaltenden Multielementoxids im Verlauf einer durch das Multielementoxid heterogen katalysierten partiellen Gasphasenoxidation von Acrolein zu Acrylsäure präventiv entgegenwirkt (d.h., das Einsetzen der Deaktivierung vergleichsweise hinauszögert).

Das Verfahren der WO 2008/104577 A1 ist dadurch gekennzeichnet, dass einem separat hergestellten pulverförmigen, Mo und V enthaltenden Multielementoxid (gleichsam als Mo-Depot) ein pulverförmiges Oxid des Molybdäns zugemischt, und das dabei resultierende Gemisch als katalytisch aktive Masse für die heterogen katalysierte partielle Gasphasenoxidation von Acrolein zu Acrylsäure eingesetzt wird (z.B. als mit Hilfe eines flüssigen Bindemittels auf die Oberfläche eines inerten geometrischen Trägerformkörpers aufgebrachte katalytisch aktive Schale).

Nachteilig an der Verfahrensweise der WO 2008/104577 A1 ist jedoch, dass bei ihrer Anwendung insbesondere das sich einstellende Aktivitätsniveau, aber auch die dabei resultierende Selektivität der Acrylsäurebildung, nicht in vollem Umfang zu befriedigen vermag.

Die Aufgabe der vorliegenden Erfindung bestand daher insbesondere darin, ein verbessertes Verfahren zur Herstellung einer katalytisch aktiven Masse, die ein Gemisch aus einem die Elemente Mo und V enthaltenden Multielementoxid und wenigstens einem Oxid des Molybdäns ist, mit der Maßgabe zur Verfügung zu stellen, dass einerseits die Deaktivierung der dabei erhältlichen katalytisch aktiven Masse (z.B. als Aktivmassenschale eines Schalenkatalysators auf die Oberfläche eines (vorzugsweise inerten) geometrischen Trägerformkörpers aufgebracht) im Rahmen einer durch sie heterogen katalysierten partiellen Gasphasenoxidation von Acrolein zu Acrylsäure erst vergleichsweise verzögert einsetzt und andererseits sowohl das dabei gezeigte Aktivitätsniveau als auch die dabei erzielte Selektivität der Acrylsäurebildung vergleichsweise erhöht ist.

Demgemäß wird ein Verfahren zur Herstellung einer katalytisch aktiven Masse, die ein Gemisch aus einem die Elemente Mo und V enthaltenden Multielementoxid und wenigstens einem Oxid des Molybdäns ist, zur Verfügung gestellt, das dadurch gekennzeichnet ist, dass das molare Verhältnis Mo /V, der im Multielementoxid enthaltenen molaren Mengen an Mo und an V, 15 : 1 bis 1 : 1 beträgt und als Verfahrensmaßnahmen

- mit Quellen der elementaren Konstituenten des Multielementoxids eine wässrige Lösung oder mit der Maßgabe eine wässrige Suspension erzeugt wird, dass jede der Quellen beim Erzeugen der wässrigen Suspension den Zustand einer wässrigen Lösung durchläuft,

- durch Sprühtrocknung der wässrigen Lösung oder der wässrigen Suspension ein Sprühpulver P erzeugt wird,

- mit dem Sprühpulver P unter Zusatz wenigstens eines pulverförmigen Oxids S des Molybdäns sowie wahlweise unter Zusatz eines oder mehrerer Formgebungshilfsmittel und nach gleichmäßiger Vermischung der genannten Bestandteile aus dem dabei resultierenden Gemisch geometrische Vorläuferformkörper geformt, wobei das wenigstens eine pulverförmige Oxid S des Molybdäns Molybdändioxid, Molybdäntrioxid oder ein Gemisch aus Molybdändioxid und Molybdäntrioxid ist, und

- die geometrischen Vorläuferformkörper unter Ausbildung der katalytisch aktiven Masse thermisch behandelt werden.

Unter einem Oxid des Molybdäns wird in dieser Schrift wie in der WO 2008/104577 A1 eine Substanz verstanden, die, abgesehen von gegebenenfalls enthaltenem Hydratwasser, das hier nicht einbezogen (nicht mitbetrachtet, außer Acht gelassen) wird, zu $\geq$ 98 Gew.- %, vorzugsweise zu $\geq$ 99 Gew.-% und besonders bevorzugt zu $\geq$ 99,5 Gew.-% bzw.

zu $\geq$ 99,9 Gew.-% und mehr nur aus Mo und O besteht (ganz besonders bevorzugt besteht es zu 100 Gew.-% aus Mo und O). D.h., ein erfindungsgemäß verwendbares Oxid S des Molybdäns, wie z.B. $MoO_2$, kann trotz der Stöchiometrie-angabe "$MoO_2$" bis zu 2 % seines Gewichts von Mo und O sowie von Wasser verschiedene Bestandteile enthalten. Im Übrigen soll der Begriff "Oxid S des Molybdäns" in dieser Schrift auch Hydrate von Oxiden des Molybdäns wie z.B. $MoO_3 \times H_2O$ subsumieren (umfassen). Diese werden häufig auch als Hydroxide formuliert. Erfindungsgemäß bevorzugt ist das Oxid S des Molybdäns jedoch frei von Hydratwasser (d.h., sein Wassergehalt liegt vorteilhaft bei < 2 Gew.-% bezogen auf die Gesamtmasse).

Besonders bevorzugt wird man für das erfindungsgemäße Verfahren als Oxid S des Molybdäns Molybdäntrioxid ($MoO_3$) verwenden. Grundsätzlich kommt als erfindungsgemäß geeignetes Molybdänoxid auch das bereits erwähnte $MoO_2$ in Betracht (vgl. z.B. "Synthese und strukturelle Untersuchungen von Molybdän-, Vanadium- und Wolframoxiden als Referenzverbindungen für die heterogene Katalyse", Dissertation von Dr. Andreas Blume, Fakultät II Mathematik und Naturwissenschaften der Technischen Universität Berlin, 2004, oder Surface Science 292 (1983) 261-6, oder J. Solid State Chem. 124 (1966) 104).

Anwendungstechnisch zweckmäßig beträgt die spezifische Oberfläche $O_M$ eines im erfindungsgemäßen Verfahren als pulverförmiges Oxid S des Molybdäns eingesetzten Molybdänoxids erfindungsgemäß bevorzugt $\leq$ 20 m²/g, besonders bevorzugt $\leq$ 15 m²/g und ganz besonders bevorzugt $\leq$ 10 m²/g. In der Regel wird die spezifische Oberfläche $O_M$ jedoch $\geq$ 0,01 m²/g, häufig $\geq$ 0,05 m²/g und vielfach $\geq$ 0,1 m²/g bzw. $\geq$ 0,5 m²/g oder $\geq$ 1 m²/g betragen (z.B. 5 m²/g). Unter der spezifischen Oberfläche wird dabei, wie stets in dieser Schrift (soweit nichts anderes explizit gesagt wird), die spezifische BET-Oberfläche verstanden (bestimmt durch Gasadsorption ($N_2$) nach Brunauer-Emmet-Teller (BET)). Eine Beschreibung der BET-Bestimmungsmethode findet sich in DIN ISO 9277 und in J. Am. Chem. Soc. Vol. 60, No. 2, Seite 309-319 (1938).

Die vorgenannten Aussagen bezüglich $O_M$ treffen insbesondere dann zu, wenn das pulverförmige Oxid S des Molybdäns pulverförmiges Molybdäntrioxid $MoO_3$ ist. Die Vorteilhaftigkeit eines vergleichsweise geringen Wertes für $O_M$ liegt darin begründet, dass ein pulverförmiges Molybdänoxid S mit einem niedrigen Wert für $O_M$ sich bei seiner alleinigen Verwendung als Aktivmasse für die entsprechende heterogen katalysierte partielle Gasphasenoxidation von Acrolein zu Acrylsäure weitgehend inert verhält, d.h., im Wesentlichen keine Umsetzung des Acroleins bedingt.

Die Körnung (Partikeldurchmesser, bzw. Partikeldurchmesserverteilung) eines in einem erfindungsgemäßen Verfahren als pulverförmiges Oxid S des Molybdäns eingesetzten Molybdänoxids ist erfindungsgemäß vorteilhaft nicht gröber als die Körnung des beim selben erfindungsgemäßen Verfahren erzeugten Sprühpulvers P (dies ermöglicht eine besonders gleichmäßige Einmischung eines pulverförmigen Oxids S des Molybdäns in ein Sprühpulver P). Dies gilt insbesondere dann, wenn das erfindungsgemäß eingesetzte pulverförmige Oxid S des Molybdäns pulverförmiges Molybdäntrioxid ($MoO_3$) ist.

Selbstredend kann die Körnung eines in einem erfindungsgemäßen Verfahren als pulverförmiges Oxid S des Molybdäns eingesetzten Molybdänoxids auch gröber oder feiner als die Körnung des beim selben erfindungsgemäßen Verfahren erzeugten Sprühpulvers P sein.

Partikeldurchmesserverteilungen sowie aus diesen entnommene Partikeldurchmesser $d_x$ (z.B. $d_{10}$, oder $d_{50}$, oder $d_{90}$) beziehen sich in dieser Schrift, soweit nicht ausdrücklich etwas anderes gesagt wird, auf Bestimmungen nach ISO 13320 mit dem Laserbeugungsspektrometer Malvern Mastersizer S (Malvern Instruments, Worcestershire WR14 1AT, United Kingdom).

Die als Messergebnis angegebenen Partikeldurchmesser $d_x$ sind dabei so definiert, dass X % des Gesamtpartikelvolumens aus Partikeln mit diesem oder einem kleineren Durchmesser bestehen.

Zur Bestimmung von Partikeldurchmesserverteilungen wird das jeweilige feinteilige Pulver anwendungstechnisch zweckmäßig über eine Dispergierrinne in den Trockendispergierer Sympatec RODOS (Sympatec GmbH, System-Partikel-Technik, Am Pulverhaus 1, D-38678 Clausthal-Zellerfeld) geführt und dort mit Druckluft trocken dispergiert und im Freistrahl in die Messzelle geblasen. In dieser erfolgt dann die eigentliche Laserbeugungsbestimmung (soweit in dieser Schrift nichts anderes explizit erwähnt wird, liegt den angegebenen $d_x$-Werten eine Bestimmung der Partikeldurchmesserverteilung bei einem Dispergierdruck (= Absolutdruck der als Treibgas angewandten Druckluft) von 2 bar abs. zugrunde).

In typischer Weise liegen die Partikeldurchmesser $d_{10}$ und $d_{90}$ eines bei einem erfindungsgemäßen Verfahren eingesetzten Sprühpulvers P im Durchmesserbereich von 1 bis 70 $\mu$m.

In dem entsprechender Weise ist die Körnung eines erfindungsgemäß mitzuverwendenden pulverförmigen Oxids S des Molybdäns (insbesondere dann, wenn es sich um Molybdäntrioxid ($MoO_3$) handelt) beim erfindungsgemäßen Verfahren in typischer Weise so beschaffen, dass sein Partikeldurchmesser $d_{90} \leq$ 20 $\mu$m, vorzugsweise $\leq$ 10 $\mu$m, besonders bevorzugt $\leq$ 5 $\mu$m und ganz besonders bevorzugt $\leq$ 3 $\mu$m oder $\leq$ 2 $\mu$m beträgt.

Normalerweise wird der Partikeldurchmesser $d_{10}$ eines erfindungsgemäß mitzuverwendenden pulverförmigen Oxids S des Molybdäns (insbesondere dann, wenn es sich um ein Molybdäntrioxid ($MoO_3$) handelt) jedoch $\geq$ 50 nm, vorzugsweise $\geq$ 75 nm, besonders bevorzugt $\geq$ 100 nm, und ganz besonders bevorzugt $\geq$ 150 nm oder $\geq$ 200 nm betragen.

D.h., für das erfindungsgemäße Verfahren kommen insbesondere solche pulverförmigen Oxide S des Molybdäns (insbesondere dann, wenn es sich um Molybdäntrioxid (MoO$_3$) handelt) in Betracht, für die 50 nm $\leq$ d$_{10}$; d$_{90}$ $\leq$ 20 $\mu$m, mit Vorteil 75 nm $\leq$ d$_{10}$; d$_{90}$ $\leq$ 10 $\mu$m, vorzugsweise 100 nm $\leq$ d$_{10}$; d$_{90}$ $\leq$ 5 $\mu$m und besonders bevorzugt 150 nm $\leq$ d$_{10}$; d$_{90}$ $\leq$ 3 $\mu$m bzw. 200 nm $\leq$ d$_{10}$; d$_{90}$ $\leq$ 2 $\mu$m beträgt.

Die Gesamteinsatzmenge an pulverförmigem Oxid S des Molybdäns (insbesondere dann, wenn es sich um Molybdäntrioxid (MoO$_3$) handelt) wird bei einem erfindungsgemäßen Verfahren, bezogen auf das Gewicht der bei diesem Verfahren erhältlichen katalytisch aktiven Masse, anwendungstechnisch zweckmäßig > 0 und $\leq$ 50 Gew.-% betragen. Erfindungsgemäß vorteilhaft wird die vorgenannte, in gleicher Weise bezogene, Gesamteinsatzmenge $\leq$ 45 Gew.-% oder $\leq$ 40 Gew.-% betragen. Erfindungsgemäß bevorzugt wird die vorgenannte, in gleicher Weise bezogene, Gesamteinsatzmenge bei $\geq$ 1 und $\leq$ 35 Gew.-%, besonders bevorzugt bei $\geq$ 3 und $\leq$ 30 Gew.-% sowie ganz besonders bevorzugt bei $\geq$ 5 und $\leq$ 30 Gew.-% bzw. bei $\geq$ 10 und $\leq$ 25 Gew.-%, oder bei $\geq$ 10 und $\leq$ 20 Gew.-% (bzw. $\leq$ 15 Gew.-%) liegen.

Die vorstehenden Gesamteinsatzmengen treffen (wie alle anderen Angaben in dieser Schrift) insbesondere dann zu, wenn es sich bei dem pulverförmigen Oxid S des Molybdäns um Molybdäntrioxid (MoO$_3$) handelt.

Grundsätzlich kann ein für das erfindungsgemäße Verfahren als pulverförmiges Oxid S des Molybdäns geeignetes Molybdänoxid (z.B. MoO$_3$) aus einer anderen Mo enthaltenden Substanz gezielt erzeugt werden.

Zu diesem Zweck kann z.B. von Ammoniumheptamolybdattetrahydrat [(NH$_4$)$_6$Mo$_7$O$_{24}$ x 4 H$_2$O] ausgegangen werden. Durch z.B. 3-stündiges thermisches Behandeln bei 350 °C im ebenfalls eine Temperatur von 350 °C aufweisenden Luftstrom wird dieses in MoO$_3$ gewandelt. Durch entsprechendes Mahlen und Sieben kann die Körnung des MoO$_3$ in beliebiger Weise bedarfsgerecht eingestellt werden. Die spezifische Oberfläche des wie beschrieben erhältlichen MoO$_3$ kann ebenfalls nach Wunsch eingestellt werden. Mit zunehmender Dauer der thermischen Behandlung und/oder Erhöhung der Temperatur der thermischen Behandlung (nach erfolgter MoO$_3$ Ausbildung unter Inertgas oder unter molekularen Sauerstoff enthaltender Gasatmosphäre (z.B. Luft)) nimmt die spezifische Oberfläche ab.

Nach wie vorstehend beschrieben vorgenommener Ausbildung des MoO$_3$ bei 350 °C ist in der Regel ein 4- bis 8-stündiges thermisches Behandeln bei 550 bis 650 °C im eine entsprechende Temperatur aufweisenden Luftstrom ausreichend, um die spezifische Oberfläche O$_M$ des MoO$_3$ auf einen Wert von $\leq$ 5 m$^2$/g oder von $\leq$ 2 m$^2$/g zu drücken. Durch Mahlen kann die spezifische Oberfläche O$_M$ eines MoO$_3$ erhöht werden.

Selbstredend können für das erfindungsgemäße Verfahren als pulverförmiges Oxid S des Molybdäns geeignete Molybdänoxide aber auch im Handel erworben oder ausgehend von im Handel erhältlichen Molybdänoxiden bedarfsgerecht präpariert werden.

Beispielsweise kann für das erfindungsgemäße Verfahren als pulverförmiges Oxid S des Molybdäns das MoO$_3$ der Climax Molybdenum Marketing Corporation (Phoenix (Arizona), USA), das einen Mo-Gehalt von 66,60 Gew.-%, eine Reinheit von $\geq$ 99,5 Gew.-% und eine spezifische Oberfläche O$_M$ von 3 $\pm$ 1 m$^2$/g aufweist (Handelsname: "pure Molybdenum Oxide Crystalline POC"), verwendet, oder durch bearbeiten desselben (z.B. Einwirkung von Ultraschall und/oder Mahlen) ein in gewünschter Weise modifiziertes, als pulverfömiges Oxid S des Molybdäns erfindungsgemäß geeignetes Molybdäntrioxid erzeugt werden.

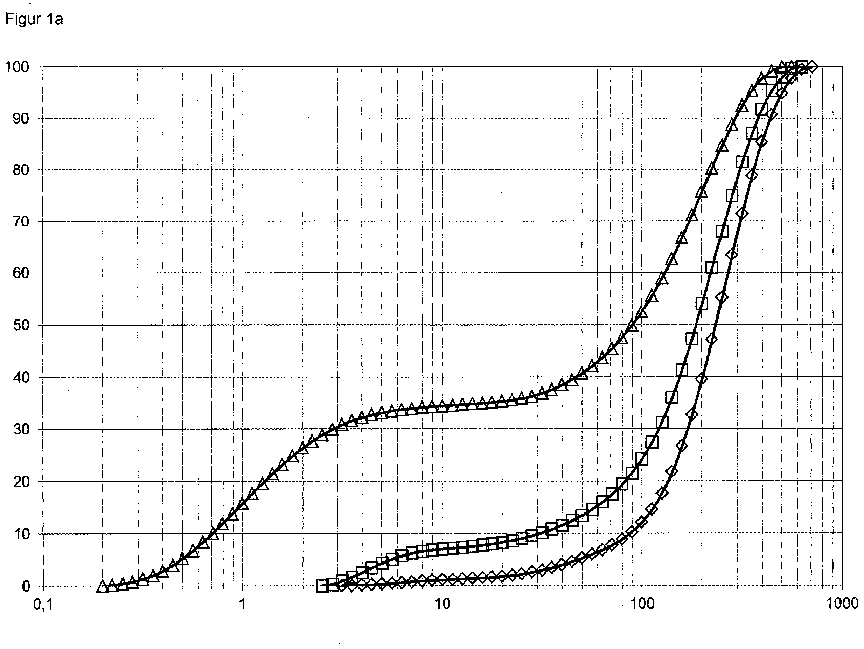

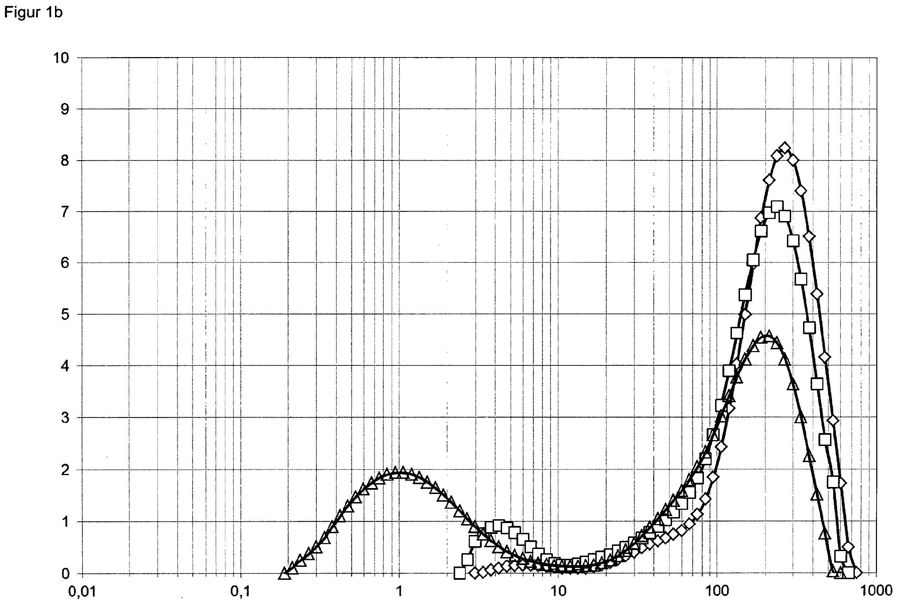

Die Figuren 1a und 1b dieser Schrift zeigen für dieses im Handel erhältliche MoO$_3$ die nach ISO 13320 in Abhängigkeit vom jeweils angewandten Dispergierdruck bestimmten Verteilungen der Partikeldurchmesser (Laser, Malvern). In der Figur 1a zeigt die Abszisse die Durchmesser [$\mu$m] im logarithmischen Maßstab (zur Basis 10) und die Ordinate zeigt den Volumenanteil des MoO$_3$ (in Vol.-%, bezogen auf das Gesamtpartikelvolumen), der den jeweiligen Partikeldurchmesser auf der Abszisse aufweist. In der Figur 1b zeigt die Abszisse wieder in logarithmischer Auftragung (im logarithmischen Maßstab zur Basis 10) den Partikeldurchmesser (die Partikelausdehnung) in $\mu$m. Die Ordinate zeigt hier jedoch den Volumenanteil des Gesamtpartikelvolumens, der den jeweiligen Durchmesser oder einen kleineren Durchmesser aufweist (angewandte Dispergierdrucke: $\diamond$ = 1,1 bar abs.; $\square$ = 2 bar abs.; $\triangle$ = 4,5 bar abs.).

Bei den Partikeln dieses käuflich zu erwerbenden MoO$_3$ handelt es sich um Agglomerate aus Primärpartikeln. Durch Einwirkung von z.B. Ultraschall kann der Zerfall der Agglomerate in die kleineren Primärpartikel bewirkt werden (vgl. Figur 1 der WO 2008/104577 A1). Für eine erfindungsgemäße Verwendung als pulverförmiges Oxid S des Molybdäns kommen grundsätzlich alle Partikeldurchmesserverteilungen in Betracht, die durch Abmischen der beiden in der Figur 1 der WO 2008/104577 A1 gezeigten Partikeldurchmesserverteilungen (Dispergierdruck: 2 bar abs.) erzeugt werden können (in beliebigen Massenverhältnissen; z.B. 1000 : 1 bis 1 : 1000, oder 100 : 1 bis 1 : 100, oder 10 : 1 bis 1 : 10, oder 5 : 1 bis 1 : 5, oder 2 : 1 bis 1 : 2). In der Praxis können diese Partikeldurchmesserverteilungen z.B. dadurch erzeugt werden, dass man Primärpartikel und Agglomerat im entsprechenden Massenverhältnis miteinander vermischt.

Ausgehend von den verschiedenen vorstehenden Partikeldurchmesserverteilungen kann durch Mahlen bzw. Mahlen und Sieben der zugehörigen pulverförmigen Molybdäntrioxide deren Zerteilungsgrad (Feinheit) beliebig gesteigert werden (können die Partikeldurchmesser zweckgemäß verringert werden). Die Fremdbestandteilspezifikation für das vorgenannte MoO$_3$ weist die Seite 8 der WO 2008/104577 aus. Selbstverständlich kann aber auch MoO$_3$ der Climax Molybdenum Marketing Corporation vom Handelstyp "POS" erfindungsgemäß als pulverförmiges Oxid S des Molybdäns eingesetzt werden.

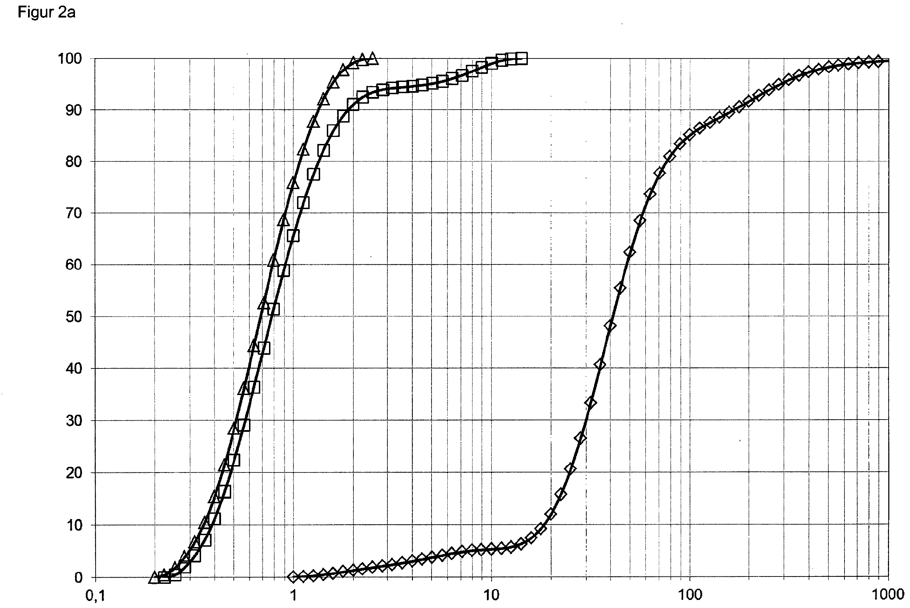

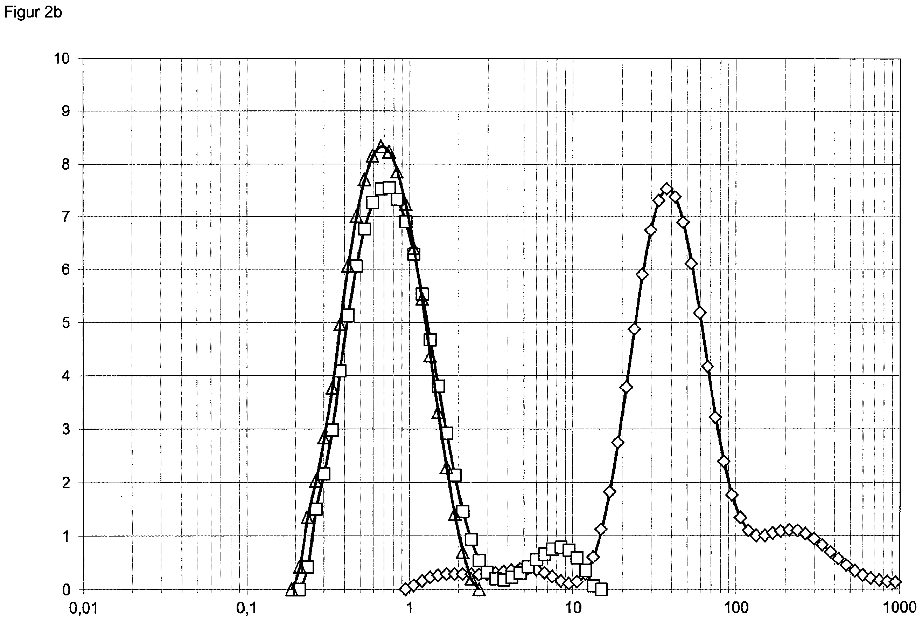

Alternativ kann als käuflich erwerbliches MoO$_3$ auch MoO$_3$ der Fa. H.C. Starck, D-38615 Goslar als pulverförmiges Oxid S des Molybdäns durch entsprechende Bearbeitung (z.B. Mahlen des käuflich zu erwerbenden Produktes) verwendet werden (Handelsname: "Molybdenum Trioxide I"). Dieses besitzt eine spezifische Oberfläche $O_M$ von 1 m$^2$/g. Der Mo-Gehalt dieses MoO$_3$ liegt bei 66,6 Gew.-%. Die Fremdkomponentenspezifikation dieses MoO$_3$ zeigt die Seite 9 der WO 2008/104577. Die zugehörige Partikeldurchmesserverteilung zeigt die Figur 2 der WO 2008/104577.

Bei den MoO$_3$-Partikeln des vorstehend beschriebenen MoO$_3$ der Fa. H.C. Starck handelt es sich ebenfalls um Agglomerate aus Primärpartikeln. Im Unterschied zu den MoO$_3$-Partikeln des vorstehend beschriebenen MoO$_3$ der Fa. Climax ist der Zusammenhalt der Primärpartikel jedoch wesentlich ausgeprägter, weshalb seitens der Anmelderin durch Einwirkung von z.B. Ultraschall kein Zerfall in die Primärpartikel bewirkt werden konnte. Durch Mahlen oder Mahlen und Sieben können die Partikeldurchmesser jedoch je nach Bedarf verringert werden.

Selbstverständlich können als pulverförmige Oxide S des Molybdäns beim erfindungsgemäßen Verfahren auch solche auf der Grundlage von Molybdenum Trioxide der "II"-Typen der Fa. H.C. Starck verwendet werden.

Im Übrigen kann für das erfindungsgemäße Verfahren aber auch pulverförmiges Oxid S des Molybdäns auf der Grundlage von MoO$_3$ der nachfolgenden Hersteller eingesetzt werden:

- Fa. Metal-Tech.-Ltd. (Israel), Reinheit > 98 Gew.-%, $O_M$ = 1,1 m$^2$/g;
- Gulf Chemical (Texas, USA), 65,76 Gew.-% Mo, $O_M$ = 1,2 m$^2$/g;
- Nanjing Chemical Industries (China), 66,6 Gew.-% Mo, $O_M$ = 0,8 m$^2$/g;
- Kankal Exports (Indien), Reinheit $\geq$ 99 Gew.-%, $O_M$ = 1,7 m$^2$/g;
- Taiyo Koko Co., Ltd. (Japan), Reinheit $\geq$ 99,7 Gew.%, $O_M$ = 1,6 m$^2$/g;
- Anhui Chizhou Huangshanling Lead and Zinc Mine (China), Reinheit $\geq$ 99,7 Gew.-%, 66,5 Gew.-% Mo, $O_M$ = 0,3 m$^2$/g; und
- CCI Moly B.V. (Niederlande), Reinheit > 99,5 Gew. %, > 66 Gew.-% Mo, $O_M$ = 2,5 m$^2$/g.

Eine für das erfindungsgemäße Verfahren besonders geeignete Partikeldurchmesserverteilung des pulverförmigen Oxids S des Molybdäns (insbesondere dann, wenn dieses ein Molybdäntrioxid (MoO$_3$) ist) zeigen die Figuren 2a und 2b dieser Schrift (dies gilt insbesondere dann, wenn das pulverförmige Oxid S des Molybdäns durch Mahlen von "pure Molybdenum Oxide Crystalline POC" der Climax Molybdenum Marketing Corporation erzeugt wurde). In Figur 2a zeigt die Abszisse die Durchmesser [$\mu$m] im logarithmischen Maßstab (zur Basis 10) und die Ordinate zeigt den Volumenanteil (Vol.-%) des Gesamtpartikelvolumens des Oxids S des Molybdäns, der, in Abhängigkeit vom angewandten Dispergierdruck, den jeweiligen Durchmesser aufweist. In der Figur 2b zeigt die Abszisse wieder in logarithmischer Auftragung (im logarithmischen Maßstab zur Basis 10) den Partikeldurchmesser (die Partikelausdehnung) in $\mu$m. Die Ordinate zeigt hier jedoch den Volumenanteil des Gesamtpartikelvolumens, der den jeweiligen Durchmesser oder einen kleineren Durchmesser aufweist. Die Bestimmungen erfolgten wie im Fall der in Figur 1 gezeigten Partikeldurchmesserverteilungen (angewandte Dispergierdrucke:

$\diamond$ = 1,1 bar abs.; $\square$ = 2 bar abs.;
$\Delta$ = 4,5 bar abs.).

Zur Herstellung des Sprühpulvers P wird beim erfindungsgemäßen Verfahren mit geeigneten Quellen der elementaren Konstituenten des die Elemente Mo und V enthaltenden Multielementoxids eine wässrige Lösung oder mit der Maßgabe eine wässrige Suspension erzeugt, dass jede der Quellen beim Erzeugen der wässrigen Suspension den Zustand einer wässrigen Lösung durchläuft.

D.h., mit Ausgangsverbindungen (Quellen), die die von Sauerstoff verschiedenen elementaren Konstituenten des in Auge gefassten Multielementoxids als Bestandteile erhalten, wird nur eine oder mehr als eine wässrige Lösung erzeugt.

Im ersteren Fall ist die wässrige Lösung unmittelbar diejenige, aus der durch Sprühtrocknung das Pulver P erzeugt wird. Die Mengen der in einer solchen wässrigen Lösung gelöst enthaltenen Ausgangsverbindungen (Quellen) sind dabei normalerweise so bemessen, dass die wässrige Lösung die relevanten elementaren Konstituenten des ins Auge gefassten Multielementoxids in der Stöchiometrie dieses Mo und V enthaltenden Multielementoxids entsprechenden molaren Mengenverhältnissen aufweist.

Im zweiten Fall, wenn mit den Ausgangsverbindungen mehr als eine wässrige Lösung erzeugt wird (wobei eine einzelne wässrige Lösung nur eine oder mehr als eine Ausgangsverbindung (Quelle) gelöst enthalten kann), werden die verschiedenen erzeugten wässrigen Lösungen miteinander vereinigt, wobei entweder eine zum Sprühpulver P sprühzutrocknende wässrige Gesamtlösung, oder eine zum Sprühpulver P sprühzutrocknende wässrige Suspension gebildet wird. Die Mengen der in der wässrigen Gesamtlösung gelöst enthaltenen Ausgangsverbindungen (Quellen) bzw. der in der wässrigen Suspension gelöst und suspendiert enthaltenen Bestandteile sind dabei normalerweise so bemessen, das die wässrige Gesamtlösung bzw. die wässrige Suspension die relevanten elementaren Konstituenten des ins Auge gefassten Multielementoxids in der Stöchiometrie dieses Mo und V enthaltenden Multielementoxids entsprechenden molaren Mengenverhältnissen aufweist.

**EP 2 953 720 B1**

Bei der erfindungsgemäßen Sprühtrocknung der wässrigen Lösung bzw. wässrigen Suspension wird die wässrige Lösung bzw. wässrige Suspension anwendungstechnisch zweckmäßig mittels einer Düse (durch Flüssigkeitsdruck, Pressluft oder Inertgas betrieben) oder mittels rotierender Zerstäuberscheiben in feine Tröpfchen zerteilt in einen heißen Gasstrom (vorzugsweise in einen heißen Luftstrom) eingebracht, der es in Bruchteilen einer Sekunde zu einem feinen Pulver trocknet. Der heiße Gasstrom (z.B. der Heißluftstrom) kann dabei grundsätzlich in Richtung mit dem Sprühstrahl (d.h. im Gleichstrom) oder gegen den Sprühstrahl (d.h. im Gegenstrom) strömen. Anwendungstechnisch günstig wird erfindungsgemäß eine Gleichstromfahrweise bevorzugt. Typische Eintrittstemperaturen des heißen Gasstroms (des vorzugsweise heißen Luftstroms; grundsätzlich kann aber auch z.B. ein heißer Stickstoffstrom, ein heißer Kohlendioxidstrom oder ein heißer Edelgasstrom verwendet werden) betragen 300 bis 360 °C, und typische Austrittstemperaturen liegen im Bereich von 100 bis 150 °C. Der Restwassergehalt des resultierenden Sprühpulvers beträgt (auf seine Gesamtmasse bezogen) erfindungsgemäß zweckmäßig < 10 Gew.-% und besonders zweckmäßig < 6 Gew.-% (geringe Restwassergehalte sind vorteilhaft). In der Regel ist der vorgenannte Restwassergehalt anwendungstechnisch pragmatisch ≥ 0,5 Gew.-%, häufig ≥ 2 Gew.-%. Angaben zu Restwassergehalten beziehen sich in dieser Schrift generell auf deren Bestimmung mit Hilfe des Mikrowellen-Analysensystems SMART System 5 der CEM GmbH in D-47475 Kamp-Lintfort. Das genannte System trocknet die zu analysierende Probe mittels fokussierter Mikrowelle (vgl. auch DE 102011084040 A1) auf der ins Messsystem eingebauten Waage. Dabei ermittelt das Analysensystem während des Trocknungsprozesses permanent den Gewichtsverlust (bis zur Gewichtskonstanz) und regelt die Trocknungsenergie am Endpunkt herunter. Eine im verwendeten Mikrowellen-Analysensystem herstellerseitig integrierte Standardluftstromführung führt entstehenden Wasserdampf kontinuierlich ab und beschleunigt den Trocknungsprozess. Ein Verbrennen oder Zersetzen der Probe wird durch Temperatursteuerung verhindert (40 °C werden nicht überschritten). Die typische Trocknungsdauer einer 0,1 g Probe liegt in der Regel bei etwa 3 Minuten.

Die Partikeldurchmesser von erfindungsgemäß einzusetzenden Sprühpulvern P liegen je nach der angewandten Tröpfchenzerteilung erfindungsgemäß zweckmäßig im Bereich von 1 μm bis 50 μm. In der Regel weisen wie beschrieben erhältliche Sprühpulver P vergleichsweise einheitliche Partikeldurchmesser auf.

Auf dem Weg vom Ort ihrer Erzeugung zur Sprühtrocknungsvorrichtung wird die sprühzutrocknende wässrige Lösung oder wässrige Suspension noch über wenigstens ein Filter geführt, um in ihr gegebenenfalls enthaltene Grobpartikel, die z.B. die Sprühdüsen verstopfen könnten, vorab ihres Eintritts in die Sprühtrocknungsvorrichtung abzutrennen. Die Temperatur der Förderleitung wird dabei anwendungstechnisch zweckmäßig auf dem Endwert der Erzeugungstemperatur der wässrigen Lösung oder der wässrigen Suspension gehalten. Die jeweils noch nicht sprühgetrocknete Restlösung bzw. Restsuspension wird durch Rühren vorteilhaft stetig durchmischt und auf der zu ihrer Sprühtrocknung relevanten Ausgangstemperatur gehalten.

Großtechnisch wird die sprühzutrocknende wässrige Lösung oder wässrige Suspension normalerweise in aus Edelstahl des Typs 1.4541 (Werkstoff 1.4541 = AISI (American Iron and Steel Institute) 321) gefertigten gerührten Behältern erzeugt. Anwendungstechnisch zweckmäßig ist die Sprühtrocknungsvorrichtung und der Rührer aus demselben Material gefertigt.

Der molare Anteil des Elements Mo an der Gesamtmenge aller von Sauerstoff verschiedenen Elemente von für das erfindungsgemäße Verfahren vorteilhaften, Mo und V enthaltenden, Multielementoxiden beträgt in der Regel 5 bis 95 mol-%, häufig 10 bis 90 mol-% und vielfach 15 bis 85 mol-% bzw. 20 bis 80 mol-%. Das molare Verhältnis von Mo zu V beträgt in für das erfindungsgemäße Verfahren günstigen Mo und V enthaltenden Multielementoxiden 15 : 1 bis 1 : 1, häufig 12 : 1 bis 2 : 1.

Neben Mo, V und O enthalten erfindungsgemäß gut geeignete Multielementoxide häufig noch wenigstens eines der Elemente Nb und W.

In vielen Fällen liegt das molare Verhältnis Mo/(Gesamtmenge aus W und Nb) in solchen Multielementoxiden im Bereich von 80 : 1 bis 1 : 4. Häufig enthalten solche für das erfindungsgemäße Verfahren gut geeigneten Multielementoxide zusätzlich noch Cu, vorzugsweise in einem molaren Verhältnis Mo/Cu im Bereich von 30 : 1 bis 1 : 3.

Neben den Elementen (elementaren Konstituenten) Nb und/oder W sowie Mo, V und O und wahlweise Cu, können für das erfindungsgemäße Verfahren geeignete Multielementoxidzusammensetzungen zusätzlich z.B. wenigstens eines der Elemente (wenigstens einen der elementaren Konstituenten) Ta, Cr, Ce, Ni, Co, Fe, Mn, Zn, Sb, Bi, Alkali (Li, Na, K, Rb, Cs), H, Erdalkali (Mg, Ca, Sr, Ba), Si, Al, Ti und Zr enthalten.

Selbstverständlich kann eine für das erfindungsgemäße Verfahren gut geeignete Multielementoxidzusammensetzung aber auch nur aus den Elementen Nb und/oder W, sowie Mo, V und O und wahlweise Cu bestehen.

Als Quellen der vorgenannten elementaren Konstituenten (als Ausgangsverbindungen, die wenigstens einen der vorgenannten elementaren Konstituenten umfassen) kommen zur Herstellung der beim erfindungsgemäßen Verfahren sprühzutrocknenden wässrigen Lösungen bzw. Suspensionen sowohl Oxide der Elemente als auch solche ein oder mehr als ein relevantes Element enthaltende Verbindungen in Betracht, die durch Erhitzen, wenigstens in Anwesenheit von Sauerstoff, in Oxide überführbar sind. Neben den Oxiden kommen daher als solche Ausgangsverbindungen (Elementquellen) vor allem Hydroxide, Halogenide, Nitrate, Formiate, Oxalate, Acetate, Carbonate und/oder deren Hydrate in Betracht.

Für das erfindungsgemäße Verfahren geeignete Ausgangsverbindungen (Quellen) der elementaren Multielementoxidkonstituenten Mo, V, W und Nb sind auch deren Oxoverbindungen (Molybdate, Vanadate, Wolframate und Niobate) bzw. die von diesen abgeleiteten Säuren. Das Element Sauerstoff enthaltende Elementquellen sind im Rahmen einer erfindungsgemäßen Herstellung von Sprühpulver P allgemein günstig.

Ist die Löslichkeit einer möglichen Elementquelle in wässrigem Medium aus sich allein heraus für die Belange des erfindungsgemäßen Verfahrens nicht ausreichend, kann z.B. der pH-Wert des wässrigen Mediums durch Zusatz entsprechender Stellmittel in geeigneter Weise modifiziert werden, um die Löslichkeit der Elementquelle im wässrigen Medium zu verbessern. Als geeignete Stellmittel kommen vor allem solche Brönstedsäuren und Brönstedbasen in Betracht, die sich unter der Einwirkung erhöhter Temperaturen, wie sie bei der thermischen Behandlung der geometrischen Vorläuferformkörper unter Ausbildung der erwünschten katalytisch aktiven Masse zur Anwendung kommen, in gasförmige Bestandteile zersetzen. Beispielhaft genannt seien als derartige pH-Stellmittel Ammoniak, Salpetersäure, Salzsäure, Essigsäure, Ameisensäure, Oxalsäure sowie Ammoniumsalze starker und schwacher Brönstedsäuren wie z.B. Ammoniumnitrat, Ammoniumchlorid, Ammoniumcarbonat, Ammoniumhydrogencarbonat, Ammoniumacetat, Ammoniumformiat und Ammoniumoxalat.

Alternativ und/oder zusätzlich können dem wässrigen Medium auch in selbigem lösliche Komplexbildner zugesetzt werden, die sich bei Einwirkung erhöhter Temperaturen (wenigstens im Beisein von molekularem Sauerstoff) zu gasförmigen Verbindungen zersetzen und/oder als gasförmige Verbindungen entweichen und in Elementquellen in ionischer Form vorliegende elementare Konstituenten zu komplexieren vermögen, was in der Regel ebenfalls zu einer Verbesserung der Löslichkeit der Elementquelle im wässrigen Medium führt. Beispielhaft genannt seien als solche Komplexbildner Ammoniak und Ethylendiamintetraessigsäure sowie deren (vorzugsweise gut wasserlösliche) Salze.

Eine weitere Maßnahme zur Verbesserung der Löslichkeit von Elementquellen in wässrigem Medium ist die Anwendung erhöhter Temperaturen. Selbstverständlich können im Rahmen der erfindungsgemäßen Verfahrensweise auch mehr als eine der verschiedenen angesprochenen Möglichkeiten zur Verbesserung der Löslichkeit von Elementquellen in wässrigem Medium simultan angewendet werden.

Auch können in die beim erfindungsgemäßen Verfahren zum Sprühpulver P sprühzutrocknende wässrige Lösung bzw. wässrige Suspension (und damit in die thermisch zu behandelnden geometrischen Vorläuferformkörper) auch andere sich bei der erfindungsgemäßen thermischen Behandlung der geometrischen Vorläuferformkörper gasförmig entweichende und/oder sich zu gasförmigen Bestandteilen zersetzende organische und/oder anorganische Materialien wie z.B. Stearinsäure, Malonsäure, Ammoniumsalze der vorgenannten Säuren, Stärken (z.B. Kartoffelstärke und/oder Maisstärke), Cellulose, gemahlene Nussschalen und/oder feinteiliges Kunststoffmehl (z.B. aus Polyethylen, Polypropylen etc.) eingearbeitet werden.

Wie im weiteren Verlauf dieser Patentanmeldung noch ausgeführt wird (und aus dem Stand der Technik im Rahmen der Herstellung üblicher Mo und V enthaltender Multielementoxidaktivmassen bekannt ist), erfolgt die erfindungsgemäße thermische Behandlung der erfindungsgemäß erzeugten geometrischen Vorläuferformkörper unter Ausbildung der katalytisch aktiven Massen vorteilhaft in einer $O_2$ und $NH_3$ aufweisenden Gasatmosphäre (vgl. WO 2008/104577 A1, WO 2004/108267 A1, EP 724481 A1 und WO 95/11081 A1). Anwendungstechnisch vorteilhaft kann sich das $NH_3$ dabei aus den Vorläuferformkörpern selbst heraus entwickeln, in dem in selbige in zweckmäßiger Weise eine entsprechende Menge an Ammoniumionen eingearbeitet wird.

Vorteilhaft lassen sich die Ammoniumionen in die erfindungsgemäß thermisch zu behandelnden geometrischen Formkörper z.B. dadurch einbringen, das man als Quellen von Elementen wie Mo, V, W oder Nb im Rahmen der Herstellung der zum Sprühpulver P sprühzutrocknenden wässrigen Lösung oder wässrigen Suspension die entsprechenden Ammoniumoxometallate solcher Elemente verwendet. Beispiele hierfür sind Ammoniummetaniobat, Ammoniummetavanadat, Ammoniummolybdat, Ammoniumheptamolybdattetrahydrat und Ammoniumparawolframatheptahydrat. Selbstverständlich können in die beim erfindungsgemäßen Verfahren zum Sprühpulver P sprühzutrocknende wässrige Lösung bzw. wässrige Suspension (und damit in die thermisch zu behandelnden geometrischen Formkörper) aber auch unabhängig von den als Quellen der Multielementoxidkonstituenten erforderlichen Ausgangsverbindungen Ammoniumlieferanten wie $NH_4NO_3$, oder $NH_4Cl$, oder Ammoniumacetat, oder Ammoniumcarbonat, oder Ammoniumhydrogencarbonat, oder $NH_4OH$, oder $NH_4CHO_2$, oder Ammoniumoxalat eingearbeitet werden.

Als Elementzusammensetzungen eines für das erfindungsgemäße Verfahren erforderlichen Mo und V enthaltenden Multielementoxids kommen insbesondere alle aus dem Stand der Technik für solche Multielementoxide bekannten Stöchiometrien in Betracht, die sich als Aktivmassen für eine katalytische Partialoxidation von Acrolein zu Acrylsäure als besonders vorteilhaft erwiesen haben.

Zu diesen Stöchiometrien zählen vor allem alle in den Schriften DE 10201002832 A1, DE 19927624 A1, WO 2011/134932 A1, WO 2008/104577 A1, DE 102012207811 A1, WO 2004/108267 A1, EP 724481 A1, WO 95/11081 A1, WO 2011/134932 A1, WO 2004/108284 A1, EP 714700 A2, DE 102005010645 A1, WO 95/11081 A1, DE 10350822 A1, US 2006/0205978 A1 und DE 102004025445 A1, und dem in diesen Schriften zitierten Stand der Technik, offenbarten (insbesondere die beispielhaft ausgeführten) Stöchiometrien.

Eine Teilmenge dieser Multielementoxidstöchiometrien genügt der nachfolgenden allgemeinen Stöchiometrie

(allgemeinen Formel) I,

$$Mo_{12}V_aX^1_bX^2_cX^3_dX^4_eX^5_fX^6_g\,O_n \qquad (I),$$

in der die Variablen folgende Bedeutung haben:

$X^1$ = W, Nb, Ta, Cr und/oder Ce,
$X^2$ = Cu, Ni, Co, Fe, Mn und/oder Zn,
$X^3$ = Sb und/oder Bi,
$X^4$ = eines oder mehrere Alkalimetalle (Li, Na, K, Rb, Cs) und/oder H,
$X^5$ = eines oder mehrere Erdalkalimetalle (Mg, Ca, Sr, Ba),
$X^6$ = Si, Al, Ti und/oder Zr,
a = 1 bis 6,
b = 0,2 bis 4,
c = 0 bis 18, vorzugsweise 0,5 bis 18,
d = 0 bis 40,
e = 0 bis 2,
f = 0 bis 4,
g = 0 bis 40, und
n = eine Zahl, die durch die Wertigkeit und Häufigkeit der von Sauerstoff verschiedenen Elemente in I bestimmt wird.

Unter diesen Multielementoxidstöchiometrien der allgemeinen Formel (I) sind für das erfindungsgemäße Verfahren jene bevorzugt, bei denen die Variablen in den folgenden Bereichen liegen:

$X^1$ = W, Nb und/oder Cr,
$X^2$ = Cu, Ni, Co und/oder Fe,
$X^3$ = Sb,
$X^4$ = Na und/oder K,
$X^5$ = Ca, Sr und/oder Ba,
$X^6$ = Si, Al und/oder Ti,
a = 2,5 bis 5,
b = 0,5 bis 2,
c = 0,5 bis 3,
d = 0 bis 2,
e = 0 bis 0,2,
f = 0 bis 1,
g = 0 bis 15, und
n = eine Zahl, die durch die Wertigkeit und Häufigkeit der von Sauerstoff verschiedenen Elemente in I bestimmt wird.

Erfindungsgemäß ganz besonders bevorzugte Multielementoxidstöchiometrien genügen der nachfolgenden allgemeinen Stöchiometrie (allgemeinen Formel) II,

$$Mo_{12}V_aX^1_bX^2_cX^5_fX^6_gO_n \qquad (II),$$

in der die Variablen folgende Bedeutung haben:

$X^1$ = W und/oder Nb,
$X^2$ = Cu und/oder Ni,
$X^5$ = Co und/oder Sr,
$X^6$ = Si und/oder Al,
a = 3 bis 4,5,
b = 1 bis 1,5,
c = 0,75 bis 2,5,
f = 0 bis 0,5,
g = 0 bis 8, und
n = eine Zahl, die durch die Wertigkeit und Häufigkeit der von Sauerstoff verschiedenen Elemente in II bestimmt wird.

An dieser Stelle sei noch festgehalten, dass sich für das erfindungsgemäße Verfahren auch Multielementoxide eignen, die als von Sauerstoff verschiedene Elemente neben den Elementen Mo und V wenigstens eines der beiden Elemente Te und Sb, und wenigstens eines der Elemente aus der Gruppe bestehend aus Nb, Pb, Ta, W, Ti, Al, Zr, Cr, Mn, Ga, Fe, Ru, Co, Rh, Ni, Pd, Pt, La, Bi, B, Ce, Sn, Zn, Si, Na, Li, K, Mg, Ag, Au und In in Kombination enthalten. Aus der letztgenannten Elementgruppe sind die Elemente Nb, Ta, W und/oder Ti bevorzugt, unter denen das Element Nb ganz besonders bevorzugt ist (vgl. z.B. WO 2004/108267 A1 und WO 2008/104577 A1).

Bevorzugte Multielementoxidstöchiometrien sind dabei diejenigen der allgemeinen Stöchiometrie III,

$$Mo_1V_bM^1_cM^2_d \qquad (III),$$

mit

M$^1$ = Te und/oder Sb,
M$^2$ = wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, Ce, Sn, Zn, Si, Na, Li, K, Mg, Ag, Au und In,
b = 0,01 bis 1,
c = > 0 bis 1, und
d = > 0 bis 1.

Bevorzugt ist M$^1$ = Te und M$^2$ = Nb, Ta, W und/oder Ti. Vorzugsweise ist M$^2$ = Nb. Der stöchiometrische Koeffizient b beträgt mit Vorteil 0,1 bis 0,6.
In entsprechender Weise beläuft sich der Vorzugsbereich für den stöchiometrischen Koeffizienten c auf 0,01 bis 1 bzw. auf 0,05 bis 0,4 und günstige Werte für d betragen 0,01 bis 1 bzw. 0,1 bis 0,6.

Besonders günstig ist es, wenn die stöchiometrischen Koeffizienten b, c und d simultan in den vorgenannten Vorzugsbereichen liegen.

An dieser Stelle sei auch noch festgehalten, dass zur Herstellung von erfindungsgemäß geeigneten Sprühpulvern P alle diejenigen Verfahrensweisen angewendet werden können, die aus dem Stand der Technik (insbesondere dem in dieser Schrift gewürdigten Stand der Technik) im Rahmen einer Herstellung von Mo und V enthaltenden Multielementoxiden vorbekannt sind.

Zur Erzeugung der beim erfindungsgemäßen Verfahren thermisch zu behandelnden geometrischen Vorläuferformkörper (unter Ausbildung der katalytisch aktiven Masse) aus dem Sprühpulver P unter Zusatz wenigstens eines Oxids S des Molybdäns, können erfindungsgemäß im Einzelnen unterschiedliche (verschiedene) Verfahrensvarianten verfolgt werden.

In einer einfachen Ausführungsform der erfindungsgemäßen Verfahrensweise werden das Sprühpulver P und das wenigstens eine pulverförmige Oxid S des Molybdäns (z.B. mit Hilfe einer Mischvorrichtung) trocken möglichst gleichmäßig miteinander vermischt. Aus dem dabei resultierenden pulverförmigen Gemisch als solchem können anschließend durch Verdichten (Pressagglomeration, Tablettieren) unmittelbar geometrische Formkörper (geometrische Vorläuferformkörper) von beliebiger gewünschter Geometrie geformt werden (z.B. wie in den Schriften DE 102008054586 A1, DE 102008040093 A1 und DE 102008040094 A1 für vergleichbare pulverförmige Gemische beispielhaft aufgezeigt). Beispiele für erfindungsgemäß typische Vorläuferformkörpergeometrien sind z.B. Kugeln (deren Durchmesser z.B. 2 bis 10 mm betragen kann), sowie Vollzylinder oder Hohlzylinder (Ringe) mit einem Außendurchmesser und einer Länge, die in typischer Weise 2 bis 10 mm betragen. Im Fall der Hohlzylinder ist eine Wandstärke von 1 bis 3 mm zweckmäßig.

Selbstverständlich können im Rahmen des Vermischens von Sprühpulver P und wenigstens einem pulverförmigen Oxid S des Molybdäns auch Hilfsmittel für die nachfolgende Formgebung (Formgebungshilfsmittel) mit eingemischt werden (vor, während und/oder nach dem Vermischen von Sprühpulver P und wenigstens einem pulverförmigen Oxid S des Molybdäns). Als solche kommen z.B. Gleit- bzw. Schmiermittel wie Graphit, Ruß, Polyethylenglykol, Stearinsäure, Salze der Stearinsäure, Stärke, Polyacrylsäure, Mineralöl, Pflanzenöl, Wasser, Bornitrid, Bortrifluorid, Glyzerin, feinteiliges Teflonpulver und/oder Celluloseether in Betracht.

Vorgenannte Gleitmittel können sich im Rahmen der erfindungsgemäßen thermischen Behandlung der geometrischen Vorläuferformkörper gegebenenfalls unter Bildung gasförmig entweichender Substanzen teilweise oder vollständig zersetzen und/oder chemisch umsetzen.

Als weitere Formgebungshilfsmittel kann das zu verdichtende Gemisch sogenannte Verstärkungsmittel zugesetzt enthalten, die den Zusammenhalt im resultierenden geometrischen Vorläuferformkörper fördern. Solche Verstärkungsmittel können beispielsweise Mikrofasern aus Glas, Asbest, Siliciumcarbid und/oder Kaliumtitanat sein.

Im Unterschied zu den Gleitmitteln bleiben Verstärkungshilfsmittel im Rahmen der erfindungsgemäßen thermischen Behandlung der geometrischen Vorläuferformkörper normalerweise im Wesentlichen vollständig erhalten.

Selbstverständlich können beim Vermischen von Sprühpulver P und wenigstens einem pulverförmigen Oxid S des Molybdäns Gleitmittel und Verstärkungsmittel auch gemeinsam mit eingemischt werden.

Bezogen auf die Gesamtmenge eines erfindungsgemäß zu Vorläuferformkörpern zu verdichtenden pulverförmigen Gemisches wird die Gesamtmenge an enthaltenen Formgebungshilfsmitteln in der Regel nicht mehr als 30 Gew.-%, meist nicht mehr als 20 Gew.-% und vielfach nicht mehr als 10 Gew.-% (häufig jedoch $\geq$ 0,1 Gew.-%, oder $\geq$ 0,2 Gew.-%, oder $\geq$ 0,5 Gew.-%, oder $\geq$ 1 Gew.-%) betragen.

Erfolgt die Formgebung bei der erfindungsgemäßen Erzeugung der geometrischen Vorläuferformkörper durch Extrudieren bzw. Strangpressen, wird beim Vermischen von Sprühpulver P und wenigstens einem pulverförmigen Oxid S des Molybdäns als Formgebungshilfsmittel erfindungsgemäß vorteilhaft wenigstens eine Flüssigkeit (ein flüssiges Bindemittel) mit eingemischt. Bei dieser handelt es sich vorzugsweise um Wasser, um eine wässrige Lösung und/oder um die Bestandteile einer wässrigen Lösung. Erfindungsgemäß vorteilhaft wird als wenigstens ein vorgenanntes flüssiges Formgebungshilfsmittel eine niedere ($C_2$- bis $C_5$-) organische Carbonsäure (z.B. Ameisensäure, Essigsäure (bevorzugt), Propionsäure, Fumarsäure und/oder Maleinsäure bzw. deren jeweilige wässrige Lösung und/oder die Bestandteile einer solchen wässrigen Lösung) eingemischt.

Gerechnet als reine niedere organische Carbonsäuren werden selbige (vorzugsweise Essigsäure) erfindungsgemäß vorteilhaft insgesamt in einer Gesamtmenge von 5 bis 10 Gew.-%, bezogen auf den Gehalt an Sprühpulver P im Gesamtgemisch, eingemischt. Der Gesamtgehalt des resultierenden Gesamtgemischs an Wasser kann z.B. 5 bis 40 Gew.-%, vorzugsweise 10 bis 30 Gew.-% betragen.

Das Einmischen einer oder mehrerer niederer organischer Carbonsäuren (vorzugsweise Essigsäure) und/oder deren wässriger Lösung erfolgt anwendungstechnisch zweckmäßig durch Kneten und möglichst homogen. Die Temperatur wird beim Kneten in der Regel nicht mehr als 50 °C betragen. In typischer Weise liegt die vorgenannte Temperatur im Bereich von 20 bis 50 °C, zweckmäßigerweise im Bereich von 30 bis 40 °C.

Die resultierende dickflüssige (breiartige) Masse (das resultierende Knetgut, die resultierende Knetmasse) wird anschließend durch Extrudieren zu Formkörpern (Vorläuferformkörpern) der gewünschten Geometrie geformt. Im einfachsten Fall können dies Stränglinge (Vollzylinder) sein. Selbstverständlich kommen erfindungsgemäß aber auch Ringe als mögliche Extrudate in Betracht.

Im Fall von durch Extrudieren erzeugten geometrischen (Vorläufer)Formkörpern schließt eine erfindungsgemäße thermische Behandlung derselben deren Trocknung mit ein. In der Regel erfolgt diese Trocknung bei Temperaturen < 200 °C, vorzugsweise $\leq$ 150 °C, üblicherweise jedoch bei Temperaturen $\geq$ 30 °C, bzw. $\geq$ 40 °C, oder $\geq$ 50 °C.

Im Übrigen umfasst (schließt ein) die erfindungsgemäße thermische Behandlung der geometrischen Vorläuferformkörper unter Ausbildung der katalytisch aktiven Masse eine solche (auch als Calcination bezeichnet) bei Temperaturen von 200 bis 600 °C, vorzugsweise von 300 bis 450 °C bzw. 300 bis 400 °C (jeweils Materialguttemperatur). Insbesondere während der Calcination weist das Materialgut erfindungsgemäß vorteilhaft eine möglichst einheitliche Temperatur auf.

Die thermische Behandlung (insbesondere die Calcination) der geometrischen Vorläuferformkörper kann sowohl unter (in) Inertgas als auch unter (in) einer oxidativen (Gas)Atmosphäre wie z.B. Luft (oder ein anderes Gemisch aus Inertgas und Sauerstoff) sowie auch unter einer reduzierend wirkenden Atmosphäre (z.B. Gemische aus Inertgas und reduzierend wirkenden Gasen wie $H_2$, $NH_3$, CO, Methan und/oder Acrolein oder die genannten reduzierend wirkenden Gase für sich) durchgeführt werden (selbstredend kann eine insgesamt reduzierend wirkende Atmosphäre auch einen begrenzten Gehalt an molekularem Sauerstoff aufweisen). Die erfindungsgemäße thermische Behandlung kann grundsätzlich aber auch unter Vakuum erfolgen.

Erfolgt die erfindungsgemäße thermische Behandlung der geometrischen Vorläuferformkörper unter gasförmiger Atmosphäre, kann diese sowohl stehen als auch fließen. Insgesamt kann die erfindungsgemäße thermische Behandlung (insbesondere die Calcination) der geometrischen (Vorläufer)Formkörper bis zu 24 h oder mehr in Anspruch nehmen. Häufig erstreckt sich die thermische Behandlung (insbesondere die Calcination) der geometrischen Vorläuferformkörper auf einen Zeitraum von Minuten bis zu einigen Stunden, z.B. von 0,5 h bis 10 h, oder von 1 h bis 5 h. Erhöhte Temperaturen gehen normalerweise mit kürzeren Zeitdauern der thermischen Behandlung (insbesondere der Calcination) einher und bei tieferen Temperaturen werden in der Regel längere Zeitdauern der thermischen Behandlung (insbesondere der Calcination) angewendet. Hohe Temperaturen und lange Behandlungsdauern (insbesondere der Calcination) mindern in der Regel die spezifische Oberfläche der im Rahmen der thermischen Behandlung der geometrischen Vorläuferformkörper (der Vorläufermasse) resultierenden katalytisch aktiven Multimetalloxidmasse.

Erfindungsgemäß zweckmäßig bleiben bei der erfindungsgemäßen thermischen Behandlung die in den geometrischen Vorläuferformkörpern enthaltenen Partikel des wenigstens einen pulverförmigen Oxids S des Molybdäns im Wesentlichen unverändert, d.h., als aufgrund ihrer spezifischen chemischen Zusammensetzung abgegrenzte Bereiche, erhalten.

Die spezifische BET-Oberfläche $O_A$ ($m^2/g$) der erfindungsgemäß erhältlichen katalytisch aktiven Massen (der (erfindungsgemäß thermisch behandelten) kalzinierten geometrischen Vorläuferformkörper) beträgt anwendungstechnisch zweckmässig 5 bis 40 $m^2/g$, vorteilhaft 10 bis 30 $m^2/g$ und vorzugsweise 10 bis 20 $m^2/g$ (z.B. 15 $m^2/g$).

Bevorzugt erfolgt die erfindungsgemäße thermische Behandlung (insbesondere die Calcination) der geometrischen Vorläuferformkörper in einer $O_2$ und $NH_3$ aufweisenden Gasatmosphäre. Das $NH_3$ kann sich dabei aus den Vorläuferformkörpern selbst heraus entwickeln, in dem in selbige eine entsprechende Menge an Ammoniumionen eingearbeitet wird.

Die resultierende katalytische Aktivität der bei der erfindungsgemäßen thermischen Behandlung anfallenden katalytisch aktiven Masse zeigt in Abhängigkeit vom Sauerstoffgehalt der Calcinationsatmosphäre in der Regel ein Optimum.

Vorzugsweise beträgt der Sauerstoffgehalt (der Gehalt an molekularem Sauerstoff) der Calcinationsatmosphäre 0,5 bis 10 Vol.-%, besonders bevorzugt 1 bis 5 Vol.-%. Sauerstoffgehalte oberhalb und unterhalb der vorgenannten Grenzen verringern die resultierende katalytische Aktivität normalerweise.

Erfindungsgemäß geeignete Calcinationsverfahren offenbaren z.B. die Schriften WO 2004/108284 A1, EP 724481 A1, WO 2008/104577 A1, WO 2004/108267 A1 und die WO 95/11081 A1, unter denen das in der letztgenannten WO-Schrift offenbarte Calcinationsverfahren erfindungsgemäß besonders bevorzugt ist.

Die im Rahmen einer erfindungsgemäßen thermischen Behandlung von geometrischen Vorläuferformkörpern anfallenden (resultierenden) geometrischen Katalysatorformkörper können als solche (als sogenannte Vollkatalysatoren) im Katalysatorfestbett zur Katalyse der heterogen katalysierten partiellen Gasphasenoxidation von Acrolein zu Acrylsäure eingesetzt werden.

Erfindungsgemäß geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Auch können die nach dem erfindungsgemäßen Verfahren erhältlichen geometrischen Katalysatorformkörper (die erfindungsgemäß erhältliche katalytisch aktive Masse; der erfindungsgemäß erhältliche Katalysator), insbesondere dann, wenn sie in einer nicht besonders einheitlichen Geometrie erzeugt wurden, in eine feinteilige Form überführt (z.B. zu Pulver oder Splitt zerkleinert) zur Katalyse einer z.B. heterogen katalysierten Partialoxidation von Acrolein zu Acrylsäure eingesetzt werden (z.B. auch im Wirbel- oder Fließbett).

Erfindungsgemäß besonders vorteilhaft wird man die erfindungsgemäß erhältliche katalytisch aktive Masse jedoch in eine feinteilige Form überführen (z.B. zu Pulver oder Splitt zerkleinert (z.B. durch Mahlen)) und diese feinteilige Form (unter Erhalt eines sogenannten Schalenkatalysators) als eine Schale aus der katalytisch aktiven Masse auf die äußere Oberfläche eines geometrischen Trägerformkörpers aufbringen.

Üblicherweise erfolgt das Aufbringen mit Hilfe eines flüssigen Bindemittels. Es fungiert als Haftflüssigkeit, mit Hilfe derer die feinteilige katalytisch aktive Masse auf die äußere Oberfläche des geometrischen Trägerformkörpers angeheftet wird. Anschließend wird die Haftflüssigkeit aus dem beschichteten geometrischen Trägerformkörper wenigstens teilweise wieder entfernt (z.B. durch Überleiten von heißem Gas, wie es in der WO 2006/094766 A1 beschrieben ist).

Als Material für die geometrischen Trägerformkörper kommen erfindungsgemäß insbesondere Aluminiumoxid, Siliciumdioxid, Silicate wie Ton, Kaolin, Steatit (vorzugsweise Steatit der Fa. CeramTec (DE) vom Typ C-220, bzw. bevorzugt mit geringem in Wasser löslichem Alkaligehalt), Bims, Aluminiumsilicat, Magnesiumsilicat, Siliciumcarbid und Zirkondioxid in Betracht. Anwendungstechnisch zweckmäßig sind die geometrischen Trägerformkörper hinsichtlich der relvanten Partialoxidation weitgehend inert (d.h., bei ihrer alleinigen Verwendung als "Katalysatoren" für die entsprechende heterogen katalysierte partielle Gasphasenoxidation von z.B. Acrolein zu Acrylsäure verhalten sie sich weitgehend inert, d.h., sie bedingen im Wesentlichen keine Umsetzung des Acroleins).

Die äußere Oberfläche des geometrischen Trägerformkörpers kann sowohl glatt als auch rau sein. Mit Vorteil ist die äußere Oberfläche des geometrischen Trägerformkörpers rau, da eine erhöhte Oberflächenrauigkeit in der Regel eine erhöhte Haftfestigkeit der aufgebrachten oxidischen Aktivmassenschale bedingt.

Als geometrische Trägerformkörper mit deutlich ausgebildeter Oberflächenrauigkeit kommen insbesondere Trägerformkörper in Betracht, die auf ihrer äußeren Oberfläche eine Splittauflage aufweisen (erfindungsgemäß bevorzugte geometrische Trägerformkörper sind Hohlzylinder mit Splittauflage auf ihrer äußeren Oberfläche).

Vorzugsweise liegt die Oberflächenrauigkeit $R_z$ der äußeren Oberfläche des geometrischen Trägerformkörpers im Bereich von 30 bis 100 $\mu$m, besonders bevorzugt im Bereich von 50 bis 70 $\mu$m (bestimmt gemäß DIN 4768 Blatt 1 mit einem "Hommel Tester für DIN-ISO-Oberflächenmessgrößen" der Fa. Hommelwerke). Besonders bevorzugt sind oberflächenraue geometrische Trägerformkörper der Fa. CeramTec aus Steatit C220.

Die Trägermaterialien können porös oder unporös sein. Vorzugsweise ist das Trägermaterial unporös (das Gesamtvolumen der Poren des geometrischen Trägerformkörpers beträgt auf das Volumen der jeweiligen geometrischen Trägerformkörpers bezogen vorteilhaft ≤ 1 Vol.-%). Die spezifische (auf die Einheit seiner Masse bezogene) BET-Oberfläche des Trägermaterials ist dementsprechend vorzugsweise gering.

Die geometrischen Trägerformkörper können regelmäßig oder unregelmäßig geformt sein, wobei regelmäßig geformte geometrische Trägerformkörper erfindungsgemäß bevorzugt werden.

Die Längstausdehnung der geometrischen Trägerformkörper liegt normalerweise im Bereich von 1 bis 10 mm (die Längstausdehnung ist die längste direkte Verbindungsgerade zweier auf der äußeren Oberfläche eines Trägerformkörpers befindlicher Punkte).

Erfindungsgemäß bevorzugt werden Kugeln oder (Voll)Zylinder, insbesondere Hohlzylinder (Ringe), als geometrische Trägerformkörper angewendet. Günstige Durchmesser für Trägerkugeln betragen 1 bis 4 mm. Werden Zylinder als geometrische Trägerformkörper verwendet, so beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Hohlzylindrische geometrische Trägerformkörper einer Länge von 3 bis 6 mm, einem Außendurchmesser von 4 bis 8 mm und einer Wanddicke von 1 bis 2 mm sind erfindungsgemäß ganz besonders bevorzugte geometrische Trägerformkörper. Als erfindungsgemäß günstige Ringgeometrien für Trägerformkörper seien beispielhaft Hohlzylinder der Geometrie 7 mm x 3 mm x 4 mm (Außendurchmesser x Länge x Innendurchmesser) sowie der Geometrie 5 mm x 3 mm x 2 mm (Außendurchmesser x Länge x Innendurchmesser) genannt. Erfindungsgemäß günstige geometrische Trägerformkörper sind auch alle in Research Disclosure Database Number 532036 im August 2008 offenbarten (insbesondere alle dort beispielhaft offenbarten) Trägerformkörper. Die in der vorliegenden Schrift offenbarte Herstellung von Schalenkatalysatoren VS und ES kann auch mit jedem dort beispielhaft offenbarten ringförmigen Trägerformkörper (insbesondere mit jenen der Geometrie 7 mm (Außendurchmesser) x 4 mm (Innendurchmesser) x 3 mm (Länge bzw. Höhe)) durchgeführt werden.

Die Dicke der auf die äußere Oberfläche der geometrischen Trägerformkörper (insbesondere der vorstehend aufgeführten ringförmigen Trägerformkörper (zu deren äußerer Oberfläche zählt auch die den Hohlraum des Rings einhüllende Oberfläche)) aufgebrachte Schale aus katalytisch aktiver Oxidmasse liegt anwendungstechnisch zweckmäßig in der Regel bei 10 bis 1000 $\mu$m. Bevorzugt beträgt diese Schalendicke bei erfindungsgemäß erhältlichen Schalenkatalysatoren 10 bis 500 $\mu$m, besonders bevorzugt 100 bis 500 $\mu$m und ganz besonders bevorzugt 200 bis 300 $\mu$m.

Mit Vorteil ist die Schalendicke über einen einzelnen Schalenkatalysator betrachtet möglichst einheitlich. Bei der Herstellung einer größeren Produktionscharge erfindungsgemäß erhältlicher Schalenkatalysatoren ist die Schalendicke über mehrere individuelle Schalenkatalysatorringkörper betrachtet ebenfalls möglichst einheitlich. Anwendungstechnisch zweckmäßig bewegt sich die vorgenannte Einheitlichkeit der Schalendicke häufig im Bereich derjenigen Angaben, die in den Ausführungsbeispielen der DE 10360058 A1 gemacht wurden.

Das Aufbringen der feinteiligen katalytisch aktiven Masse auf die äußere Oberfläche des geometrischen Trägerformkörpers kann beispielsweise dadurch erfolgen, dass zunächst die äußere Oberfläche mit dem flüssigen Bindemittel kontrolliert befeuchtet (z.B. durch Besprühen) wird. Durch in Kontakt bringen der so befeuchteten geometrischen Trägerformkörper mit der feinteiligen erfindungsgemäß erhaltenen katalytisch aktiven oxidischen Masse wird nachfolgend eine Schicht der Aktivmasse auf der befeuchteten Oberfläche angeheftet (z.B. die befeuchteten geometrischen Trägerformkörper wie in der EP 714700 A2 beschrieben mit der feinteiligen katalytisch aktiven Masse (mit dem Aktivmassenpulver) bestäuben).

"Kontrolliert befeuchten" heißt in diesem Zusammenhang, dass man die Trägeroberfläche zweckmäßig so befeuchtet, dass diese zwar flüssiges Bindemittel absorbiert aufweist, aber auf der Trägeroberfläche keine Flüssigphase als solche visuell in Erscheinung tritt.

Ist die Trägeroberfläche zu feucht, agglomeriert die feinteilige katalytisch aktive Multimetalloxidmasse zu getrennten Agglomeraten, anstatt auf die Oberfläche aufzuziehen. Detaillierte Angaben hierzu finden sich in der DE 2909671 A1 und in der DE 10051419 A1, sowie in der EP 714700 A2. Selbstredend kann man den Vorgang zur Erzielung einer erhöhten Schichtdicke periodisch wiederholen. In diesem Fall wird der beschichtete Grundkörper zum neuen "Trägerkörper" etc..

Es können aber auch alle anderen in der EP 714700 A2 als Stand der Technik gewürdigten Aufbringungsverfahren zur Herstellung der vorstehend ausgeführten Schalenkatalysatoren angewendet werden.

Als flüssiges Bindemittel kommt z.B. Wasser, ein organische Lösungsmittel oder eine Lösung einer organischen Substanz (z.B. eines organischen Lösungsmittels) in Wasser, oder in einem organischen Lösungsmittel, oder in einer wässrigen Lösung eines organischen Lösungsmittels in Betracht. Beispielhaft genannt seien als organische Bindemittel ein- oder mehrwertige organische Alkohole wie z.B. Ethylenglykol, 1,4-Butandiol, 1,6-Hexandiol oder Glyzerin, ein- oder mehrwertige organische Carbonsäuren wie Propionsäure, Oxalsäure, Malonsäure, Glutarsäure oder Maleinsäure, Aminoalkohole wie Ethanolamin oder Diethanolamin sowie ein- oder mehrwertige organische Amide wie z.B. Formamid. Als in Wasser, in einer organischen Flüssigkeit oder in einem Gemisch aus Wasser und einer organischen Flüssigkeit lösliche organische Bindemittelbestandteile (Bindemittelpromotoren) sind z.B. Monosaccharide und Oligosaccharide wie Glucose, Fructose, Saccharose und/oder Lactose geeignet.

Besonders vorteilhaft wird als flüssiges Bindemittel eine Lösung bestehend aus 20 bis 90 Gew.-% Wasser und 10 bis 80 Gew.-% einer organischen Verbindung verwendet. Vorzugsweise beträgt der organische Anteil an den vorgenannten flüssigen Bindemitteln 10 bis 50 und besonders bevorzugt 20 bis 30 Gew.-%. Ganz besonders bevorzugte flüssige Bindemittel sind Lösungen, die aus 20 bis 90 Gew.-% Wasser und 10 bis 80 Gew.-% Glyzerin bestehen. Mit Vorteil beträgt der Glyzerinanteil in diesen wässrigen Lösungen 10 bis 50 Gew.-% und besonders bevorzugt 20 bis 30 Gew.-%. Die Vorteilhaftigkeit von erfindungsgemäß bevorzugten Bindemitteln liegt unter anderem darin begründet, dass sie sowohl die feinteilige katalytisch aktive Masse (bzw. die feinteilige Vorläufermasse (siehe weiter unten)) als auch die äußere Oberfläche der geometrischen Trägerformkörper in voll befriedigender Weise zu benetzen vermögen.

Die Feinheit der auf der äußeren Oberfläche des geometrischen Trägerformkörpers aufzubringenden feinteiligen katalytisch aktiven (oxidischen) Masse (oder deren Vorläufermasse (siehe weiter unten)) wird selbstredend an die gewünschte Schalendicke angepasst. Für den Schalendickenbereich von 100 bis 500 μm eignen sich z.B. solche Aktivmassenpulver, von denen wenigstens 50 % der Gesamtzahl der vorzugsweise körnigen Pulverpartikel ein Sieb der Maschenweite (kreisförmige Maschen) 1 bis 20 μm bzw. alternativ 1 bis 10 μm passieren und deren numerischer Anteil an Partikeln mit einer Längstausdehnung oberhalb von 50 μm (an Partikeln, die ein Sieb der Maschenweite (kreisförmige Maschen) 50 μm nicht mehr passieren) weniger als 10 % beträgt. Im Übrigen gilt das auf Seite 18 der WO 2005/120702 A1 Gesagte in entsprechender Weise.

Erfindungsgemäß bevorzugt wird man wie beschrieben Schalenkatalysatoren gemäß der in der EP 714700 A2 beschriebenen und beispielhaft ausgeführten (siehe auch WO 2011/134932 A1 sowie die Ausführungsbeispiele der DE 10360057 A1) Herstellweise erzeugen. Eine wässrige Lösung aus 75 Gew.-% Wasser und 25 Gew.-% Glyzerin ist das bevorzugte flüssige Bindemittel. Das erfindungsgemäße Verfahren der thermischen Behandlung der geometrischen Vorläuferformkörper wird man erfindungsgemäß vorteilhaft gemäß der in der DE 10360057 A1 beschriebenen und beispielhaft ausgeführten Verfahrensweise durchführen.

Die erfindungsgemäße Verfahrensweise umfasst aber auch solche Verfahren zur Herstellung einer katalytisch aktiven Masse, bei denen die Formung von geometrischen (Vorläufer)Formkörpern mit dem aus einem Sprühpulver P, wenigstens einem pulverförmigen Oxid S des Molybdäns sowie wahlweise einem oder mehreren Formgebungshilfsmitteln bestehenden (feinteiligen) Gemisch so erfolgt, dass man (in entsprechender Weise wie für das Aufbringen einer Aktivmassenschale beschrieben) unmittelbar von diesem (feinteiligen) Gemisch (von der feinteiligen Vorläufermasse) als solchem eine Schale auf die äußere Oberfläche eines geometrischen Trägerformkörpers aufbringt. Im Rahmen der erfindungsgemäßen thermischen Behandlung der so erzeugten geometrischen (Vorläufer)Formkörper (die auch die wenigstens teilweise Entfernung des zur Aufbringung mitverwendeten flüssigen Bindemittels umfasst) werden unmittelbar erfindungsgemäße Schalenkatalysatoren erhalten, bei denen eine Schale aus katalytisch aktiver Masse auf der äußeren Oberfläche eines (katalytisch im Wesentlichen inerten) geometrischen Trägerformkörpers aufgebracht ist.

Wie bereits erwähnt, eignen sich erfindungsgemäß erhältliche katalytisch aktive Massen insbesondere zur Katalyse einer heterogen katalysierten partiellen Gasphasenoxidation von Acrolein zu Acrylsäure (z.B. einer solchen, wie sie in den Schriften WO 2007/082827 A1, WO 2004/085365 A2, WO 2004/085367 A1, WO 2004/085368 A2, WO 2004/085369 A1, WO 2004/085370 A1, WO 2005/016861 A1, WO 2005/047226 A1 und WO 2005/042459 A1 beschrieben ist). Sie zeichnen sich dabei insbesondere dadurch aus, dass ein mit ihnen beschicktes Katalysatorbett bei der Durchführung der Partialoxidation eine hohe Standzeit aufweist, während der die Zielproduktbildung bei hoher Aktivität mit hoher Selektivität erfolgt. Die bevorzugte Anwendungsform einer erfindungsgemäß erhältlichen katalytisch aktiven Masse ist dabei diejenige eines Schalenkatalysators, der vorzugsweise eine ringförmige Geometrie aufweist (besonders bevorzugt wird dabei der im Beispiel der vorliegenden Schrift beispielhaft ausgeführte Schalenkatalysator eingesetzt (z.B. in allen Ausführungsbeispielen und in allen Vergleichsbeispielen der vorstehenden WO-Schriften (WO 2007/082827 A1, WO 2004/085365 A2, WO 2004/085367 A1, WO 2004/085368 A2, WO 2004/085369 A1, WO 2004/085370 A1, WO 2005/016861 A1, WO 2005/047226 A1 und WO 2005/042459 A1), in denen er den dort jeweils eingesetzten Katalysator zu ersetzen vermag; das ebenda für den Schalenkatalysator aus dem Beispiel der vorliegenden Schrift Gesagte gilt auch für den Schalenkatalysator aus dem Vergleichsbeispiel der vorliegenden Schrift)).

Grundsätzlich eignen sich erfindungsgemäß erhältliche katalytisch aktive Massen in entsprechend vorteilhafter Weise aber auch zur Katalyse der heterogen katalysierten partiellen Gasphasenoxidation von Methacrolein zu Methacrylsäure.

Das Vorstehende gilt vor allem dann, wenn die heterogen katalysierte partielle Gasphasenoxidation von Acrolein bzw. Methacrolein (d.h., abgekürzt geschrieben von "(Meth)acrolein") zu Acrylsäure bzw. Methacrylsäure (d.h., abgekürzt geschrieben zu "(Meth)acrylsäure") bei hohen (Meth)acroleinlasten durchgeführt wird, wie es z.B. die DE 10307983 A1, die DE 19948523 A1, die DE 19910508 A1, die WO 2008/104577 A1, die WO 2011/134932 A1, die DE 19927624 A1 und die DE 10360057 A1 beschreiben.

Die heterogen katalysierte partielle Gasphasenoxidation kann dabei in an sich bekannter Weise durchgeführt werden. D.h., es wird ein das (Meth)acrolein, molekularen Sauerstoff und wenigstens ein inertes Verdünnungsgas enthaltendes Reaktionsgasgemisch bei erhöhter Temperatur durch ein Katalysatorbett geführt, dessen Katalysatoren als Aktivmasse wenigstens eine erfindungsgemäß erhältliche katalytisch aktive Masse aufweisen, und während der Verweilzeit des (Meth)acroleins im Katalysatorbett erfolgt die Umsetzung desselben zur (Meth)acrylsäure.

Bevorzugt ist als Katalysatorbett ein Katalysatorfestbett. Grundsätzlich kommen für das Verfahren aber auch ein Wirbelbett oder ein Fließbett in Betracht. In der Regel führt Wasserdampf als Bestandteil des Reaktionsgasgemischs zu einer Verbesserung von Selektivität und Aktivität. Im Übrigen sind inerte Verdünnungsgase mit erhöhter molarer spezifischer Wärme wie z.B. n-Propan oder $CO_2$ von Vorteil. Es handelt sich dabei um solche Gase, die sich beim Durchgang des Reaktionsgasgemischs durch das Katalysatorbett zu ≤ 5 mol-%, vorzugsweise zu ≤ 3 mol-% und besonders bevorzugt zu ≤ 1 mol-% bzw. überhaupt nicht chemisch verändern.

Zur Durchführung der Gasphasenpartialoxidation des (Meth)acroleins eignen sich insbesondere Wärmeaustauscherreaktoren. Ein Wärmeaustauscherreaktor weist wenigstens einen Primärraum und wenigstens einen Sekundärraum auf, die beide durch eine Trennwand voneinander getrennt sind. In dem wenigstens einen Primärraum wird die Katalysatorbeschickung platziert, die wenigstens eine erfindungsgemäß erhältliche katalytisch aktive Masse umfasst und von einem (Meth)acrolein enthaltenden Reaktionsgasgemisch durchströmt wird. Gleichzeitig wird der Sekundarraum von einem fluiden Wärmeträger durchströmt und durch die Trennwand hindurch findet zwischen den beiden Räumen der Wärmeaustausch statt, der den Zweck verfolgt, die Temperatur des Reaktionsgasgemischs auf dessen Weg durch das Katalysatorbett zu kontrollieren und zu steuern.

In der Regel wird die Gasphasenpartialoxidation des (Meth)acroleins in einem eine oder mehrere Temperaturzonen aufweisenden Rohrbündel(wärmeaustauscher)reaktor durchgeführt, wie es z.B. die EP 700174 A1, die EP 700893 A1, die DE 19910508 A1, die DE 19948523 A1, die DE 19910506 A1, die DE 19948241 A1, die DE 2830765 A1, die DE 2513405 A1, die US 3147084 A, die DE 2201428 A1, die EP 383224 A2, die JP 2007-260588 A und die JP 58096041 A beschreiben.

Ein Katalysatorfestbett befindet sich dabei in Form einer entsprechenden Schüttung von Katalysatorformkörpern (gegebenenfalls im Gemisch mit verdünnend wirkenden inerten geometrischen Formkörpern) in den Metallrohren (Kontaktrohren) des Rohrbündelreaktors und um die Metallrohre werden das oder die Temperaturmedien geführt (bei mehr als einer Temperaturzone wird eine entsprechende Anzahl räumlich im Wesentlich getrennter Temperaturmedien um die Metallrohre geführt). Das Temperaturmedium ist in der Regel eine Salzschmelze. Durch die Kontaktrohre wird das Reaktionsgasgemisch geführt.

Alternativ kann sich das Katalysatorfestbett z.B. auch in den Räumen zwischen von einem Wärmeträger durchströmten Thermoblechplatten eines Thermoblechplattenreaktors befinden, wie es z.B. die DE 10 2004 017 150 A1, die DE 19952964 A1 und die DE 10361456 A1 empfehlen.

Das Katalysatorfestbett kann, wie bereits gesagt, ganz allgemein nur aus erfindungsgemäß erhältlichen Katalysatoren, aber auch aus solchen Katalysatoren mit inerten geometrischen Formkörpern verdünnt bestehen. Als inerte geometrische Formkörper können dabei z.B. die zur Herstellung Schalenkatalysatoren verwendeten geometrischen Trägerformkörper (Trägerkörper) eingesetzt werden. Vor und/oder hinter dem Katalysatorfestbett kann sich eine reine Inertformkörperschüttung befinden (derartige reine Inertformkörperschüttungen werden bei der Berechnung der Belastung des Katalysatorfestbetts mit Reaktionsgas bzw. mit einer Reaktionsgaskomponente normalerweise nicht miteinbezogen).

In einem Rohrbündelreaktor eingesetzte Kontaktrohre sind üblicherweise aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 29 mm oder 21 bis 26 mm. Ihre Länge beträgt zweckmäßig 2 bis 4 m.

Anwendungstechnisch zweckmäßig beläuft sich die im Rohrbündelbehälter untergebrachte Anzahl an Kontaktrohren auf wenigstens 5000, vorzugsweise auf wenigstens 10 000. Häufig beträgt die Anzahl der im Reaktorbehälter untergebrachten Kontaktrohre 15 000 bis 40 000. Rohrbündelreaktoren mit einer oberhalb von 50 000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrleitung) 35 bis 45 mm beträgt (vgl. z.B. EP-B 468290 A1).

Als Wärmeaustauschmittel für Rohrbündelreaktoren besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

Eine Beschickung von Kontaktrohren in Rohrbündelreaktoren mit erfindungsgemäß erhältlichen (insbesondere dem im Beispiel (aber auch dem im Vergleichsbeispiel) der vorliegenden Schrift exemplarisch ausgeführten) Katalysatoren ist vor allem dann vorteilhaft, wenn der Rohrbündelreaktor bei einer (Meth)acroleinbelastung der Katalysatorbeschickung betrieben wird, die > 130 Nl/l•h, oder ≥ 150 Nl/l•h, oder ≥ 160 Nl/l•h, oder ≥ 170 Nl/l•h, oder ≥ 180 Nl/l•h, oder ≥ 200 Nl/l•h, oder ≥ 220 Nl/l•h, oder ≥ 240 Nl/l•h, oder ≥ 260 Nl/l•h beträgt. Selbstredend ist eine solche Katalysatorbeschickung auch bei kleineren (z.B. ≤ 130 Nl/l•h, oder ≤ 100 Nl/l•h, oder ≤ 80 Nl/l•h, oder ≤ 60 Nl/l•h) (Meth)acroleinbelastungen vorteilhaft.

In der Regel wird die (Meth)acroleinbelastung der Katalysatorbeschickung ≤ 400 Nl/l•h, oder ≤ 350 Nl/l•h, oder ≤ 300 Nl/l•h, oder ≤ 280 Nl/l•h betragen (entsprechende Belastungen können auch in Thermoblechplattenreaktoren realisiert werden).

Unter der Belastung eines Katalysatorfestbetts mit Reaktionsgaseingangsgemisch wird in dieser Schrift die Menge an Reaktionsgaseingangsgemisch in Normlitern (= Nl; das Volumen in Litern, das die entsprechende Reaktionsgaseingangsgemischmenge bei Normalbedingungen, d.h., bei 0 °C und 1 atm (1,01 bar) einnehmen würde) verstanden, die dem Katalysatorfestbett, bezogen auf das Volumen seiner Schüttung (Schüttungsabschnitte aus reinem Inertmaterial werden dabei in das Volumen der Schüttung nicht mit einbezogen; im Übrigen ist das Volumen einer Schüttung das Volumen des von der Schüttung (bzw. von deren relevanten Schüttungsabschnitten) eingenommenen Leerraums), d.h., auf sein Schüttvolumen bezogen, pro Stunde zugeführt wird (--> Einheit = Nl/l•h).

Die Belastung kann auch nur auf einen Bestandteil des Reaktionsgaseingangsgemischs bezogen sein (z.B.

nur auf die partiell zu oxidierende organische Ausgangsverbindung). Dann ist sie in entsprechender Weise die Volumenmenge dieses Bestandteils (z.B. der organischen Ausgangsverbindung der Partialoxidation) in Normlitern, die dem Katalysatorfestbett, bezogen auf das Volumen seiner Schüttung (Schüttungsabschnitte aus reinem Inertmaterial werden dabei in das Volumen der Schüttung nicht mit einbezogen; im Übrigen ist das Volumen einer Schüttung das Volumen des von der Schüttung (bzw. von deren relevanten Schüttungsabschnitten) eingenommenen Leerraums), pro Stunde zugeführt wird (--> Einheit = Nl/l•h).

Die volumenspezifische Aktivität des Katalysatorfestbetts wird man dabei in der Regel so gestalten, dass sie in Strömungsrichtung des Reaktionsgases zunimmt.

Die ist in einfacher Weise z.B. dadurch zu realisieren, dass man in Strömungsrichtung des Reaktionsgases den Verdünnungsgrad des Katalysatorfestbetts mit inerten Formkörpern abnehmend gestaltet.

Im Übrigen kann man die heterogen katalysierte Partialoxidation mit z.B. erfindungsgemäß erhältlichen Schalenkatalysatoren in allen Aspekten ganz generell so durchführen, wie es z.B. die DE A 10350822 A1 ausführt. Der (Meth)acroleingehalt im Reaktionsgaseingangsgemisch kann z.B. bei Werten von 3 oder 6 bis 15 Vol.-%, häufig bei 4 oder 6 bis 10 Vol.-%, bzw. 5 bis 8 Vol.-% liegen (jeweils bezogen auf das Gesamtvolumen des Reaktionsgaseingangsgemischs).

Das molare Verhältnis von $O_2$: (Meth)acrolein im Reaktionsgaseingangsgemisch wird normalerweise $\geq 1$ betragen. Üblicherweise wird dieses Verhältnis bei Werten $\leq 3$ liegen. Vielfach wird man die heterogen katalysierte (Meth)acroleinpartialoxidation zu (Meth)acrylsäure mit einem im Reaktionsgaseingangsgemisch vorliegenden (Meth)acrolein : Sauerstoff: Wasserdampf: Inertgas-Volumenverhältnis (Nl) von 1 : (1 bis 3) : (0 bis 20) : (3 bis 30), vorzugsweise von 1 : (1 bis 3) : (0,5 bis 10) : (7 bis 10) ausführen.

Als inerte Verdünnungsgase (dies sind Gase oder Gemische aus solchen Gasen, die beim einmaligen Durchgang des Reaktionsgasgemischs durch das Katalysatorbett (z.B. ein Katalysatorfestbett) zu wenigstens 95 mol-%, vorzugsweise zu wenigstens 97 mol-% oder zu wenigstens 99 mol-%, und am besten zu 100 mol-% chemisch unverändert erhalten bleiben) kommen dabei u.a. $N_2$, $CO_2$, CO, Edelgase, Propan, Ethan, Methan, Butan und/oder Pentan (d.h., jedes als alleiniges Verdünnungsgas oder im Gemisch mit einem anderen oder mit mehreren anderen dieser inerten Verdünnungsgase) in Betracht. Die Reaktionstemperaturen einer solchen heterogen katalysierten (Meth)acroleinpartialoxidation liegen dabei üblicherweise im Bereich von 200 bis 400 °C, in der Regel von 220 bis 380 °C, vielfach von 230 bis 350 °C, häufig von 245 bis 285 °C bzw. von 245 bis 265 °C. Der Arbeitsdruck (Absolutdruck) beträgt normalerweise 101,3 bis 350 kPa, bzw. 101,3 bis 250 kPa, oder 101,3 bis 205 kPa (insbesondere als Eingangsdruck ins Katalysatorfestbett). Selbstverständlich kann die (Meth)acroleinpartialoxidation mit den erfindungsgemäß erhältlichen Katalysatoren aber auch bei unterhalb des Atmosphärendrucks liegenden Arbeitsdrucken durchgeführt werden.

Der (Meth)acroleinumsatz, bezogen auf einen einmaligen Durchgang des Reaktionsgasgemischs durch das z.B. Katalysatorfestbett, beträgt üblicherweise $\geq 90$ mol-%, häufig $\geq 98$ mol-%, und vielfach $\geq 99$ mol-%, oder sogar $\geq$ 99,9 mol-%.

Im Übrigen kann das Partialoxidationsverfahren in völliger Entsprechung zu den Empfehlungen der Lehren der DE 10 2007 019 597 A1 bzw. der WO 2008/104577 A1, oder der WO 2011/134932 A1 ausgeführt werden.

Insbesondere kann als Quelle des für die Partialoxidation benötigten (Meth)acroleins unmittelbar das (Meth)acrolein enthaltende Produktgasgemisch einer heterogen katalysierten Partialoxidation einer $C_3$-/$C_4$-Vorläuferverbindung (z.B. Propen oder Isobuten) des (Meth)acroleins zu (Meth)acrolein verwendet werden, ohne dass aus einem solchen Produktgasgemisch das (Meth)acrolein vorab abgetrennt werden muss.

Unter der Selektivität S der (Meth)acrylsäurebildung (mol-%) wird in dieser Schrift verstanden:

$$S = \frac{\text{Molzahl (Meth)acrolein umgesetzt zu (Meth)acrylsäure x 100 mol-\%}}{\text{Molzahl (Meth)acrolein insgesamt umgesetzt}}$$

(die Umsatzzahlen sind dabei jeweils auf einen einmaligen Durchgang des Reaktionsgasgemischs durch das Katalysatorbett bezogen). Ein Vergleich von Selektivitäten S einer Zielproduktbildung bezieht sich in zweckmäßiger Weise auf gleiche Eduktumsätze.

Eine unter ansonsten unveränderten Reaktionsbedingungen bei tieferer Temperatur zum gleichen Umsatz führende Aktivmasse (führender Katalysator) verfügt über eine höhere Aktivität. Der Umsatz U an (Meth)acrolein (mol-%) ist in entsprechender Weise definiert als:

$$U = \frac{\text{Molzahl umgesetztes (Meth)acrolein}}{\text{Molzahl eingesetztes (Meth)acrolein}} \text{ x 100 mol-\%.}$$

Die Abtrennung der (Meth)acrylsäure aus dem Produktgasgemisch der Partialoxidation kann in an sich bekannter Weise z.B. dadurch erfolgen, dass die (Meth)acrylsäure zunächst durch absorptive und/oder kondensative Maßnahmen in die kondensierte Phase überführt wird. Durch daran anschließende thermische Trennverfahren wie z.B. Rektifikation und/oder Kristallisation, kann aus der kondensierten Phase anschließend (Meth)acrylsäure in beliebiger Reinheit isoliert werden (vgl. z.B. DE 602004924 T2 und WO 2006/114428 A1 sowie der in diesen Schriften zitierte Stand der Technik).

Beispiel und Vergleichsbeispiel

A) Herstellung eines ringförmigen Vergleichsschalenkatalysators VS

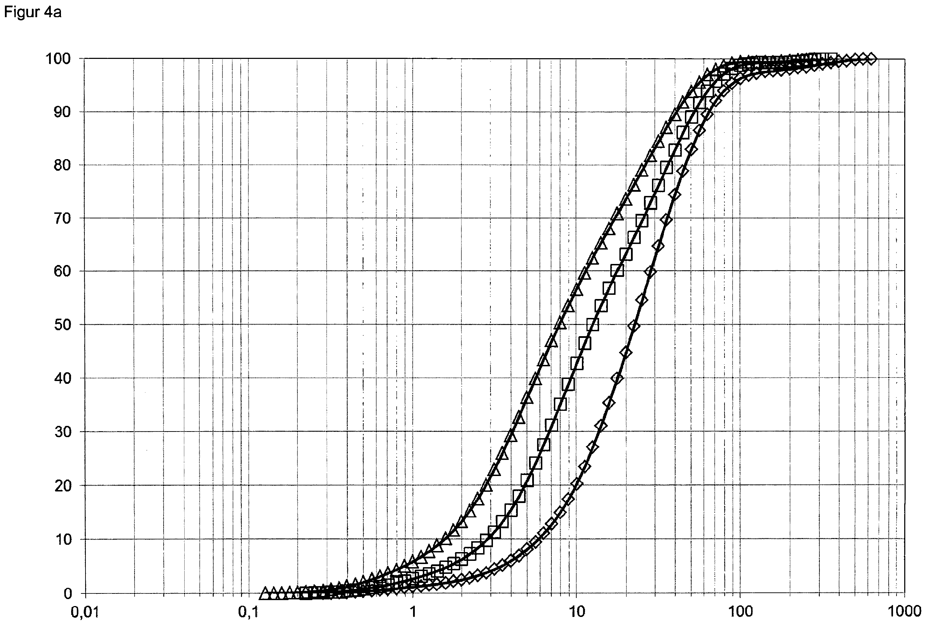

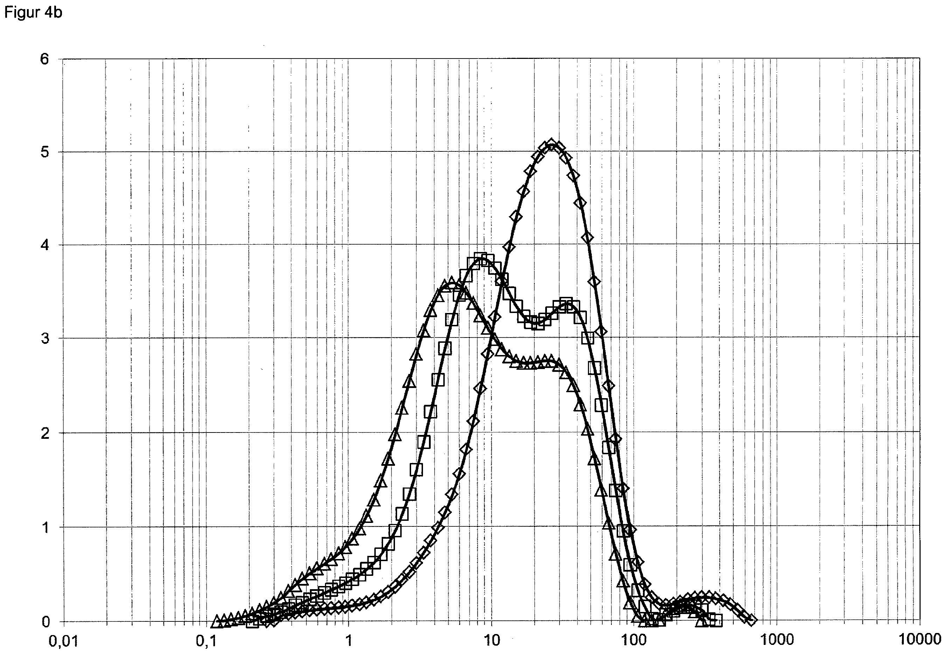

Wie im Ausführungsbeispiel 1A der WO 2011/134932 A1 beschrieben, wurde eine katalytisch aktive Multielementoxidmasse der Stöchiometrie $Mo_{12}V_3W_{1,2}Cu_{1,2}O_x$ hergestellt (vgl. Seite 9 der WO 2011/134932; die Größenverteilung der Partikel des dabei erzeugten Sprühpulvers zeigen die Figuren 4a und 4b dieser Anmeldung (der vorliegenden Schrift) in Abhängigkeit vom Dispergierdruck der zur Trockendispergierung verwendeten Druckluft ($\diamond$ = 1,1 bar abs.; $\square$ = 2,0 bar abs.; $\Delta$ = 4,5 bar abs.); der Restwassergehalt des Sprühpulvers war dabei 4,2 Gew.-% bezogen auf das Gewicht des Sprühpulvers; das Knetgut wurde dabei wie in B) der vorliegenden Schrift extrudiert und die Stränglinge wie in B) der vorliegenden Schrift im Umlufttrockenschrank getrocknet; der Restwassergehalt der getrockneten Stränglinge betrug (bezogen auf ihr Gewicht) 1,97 Gew.-%; der Glühgewichtsverlust der getrockneten Stränglinge war (bezogen auf ihr Gewicht) 22,15 Gew.% (zur Glühgewichtsverlustmessung wurde, wie stets in der vorliegenden Schrift, eine 15 g Probe der getrockneten Stränglinge in einen auf 25°C abgekühlten und zuvor bei 600°C an Luft während 3 h ausgeglühten Keramiktiegel gefüllt; der Keramiktiegel wurde dann im Beisein von Luft in einen Muffelofen (Typ: LE 14/11/P300, Innenvolumen = 20 l) der Firma Nabertherm Schweiz AG in CH-4614 Hägendorf gegeben; der Muffelofen wurde verschlossen und das enthaltene Glühgut ohne weitere Luftzufuhr zunächst innerhalb von 1 h linear von 25 °C auf 500 °C erhitzt; anschließend wurde die Glühguttemperatur von 500°C während 3 h aufrechterhalten und nachfolgend der Muffelofen und das in selbigem enthaltene Glühgut durch sich selbst überlassen innerhalb von 5 h im Wesentlichen linear auf 25 °C abgekühlt; die Differenz der Probengewichte vor dem Glühen und nach dem Glühen, bezogen auf das Probengewicht vor dem Glühen, ergibt den Glühgewichtsverlust in Gew.-%).

Die dem Drehrohrofen entnommene katalytisch aktive Multielementoxidmasse wurde anschließend in einer Mühle ZM 200 der Fa. Retsch (Rotortyp: 24-Zahn-Rotor; Siebgrösse (Trapezlochung): 0,12 mm; 18000 Umdrehungen pro Minute) zu einem feinteiligen Pulver gemahlen, von dem 50 % der Pulverpartikel ein Sieb der Maschenweite (kreisförmige Maschen) 1 bis 10 $\mu$m passierten und dessen numerischer Anteil an Partikeln mit einer Längstausdehnung oberhalb von 50 $\mu$m (an Partikeln, die ein Sieb der Maschenweite (kreisförmige Maschen) 50 $\mu$m nicht mehr passierten) weniger als 1 % betrug.

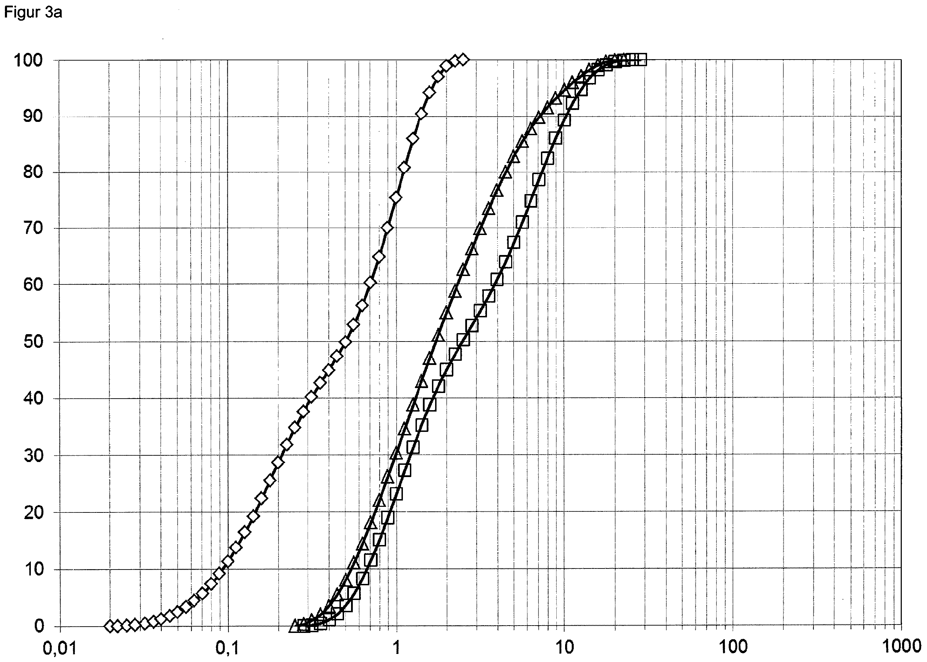

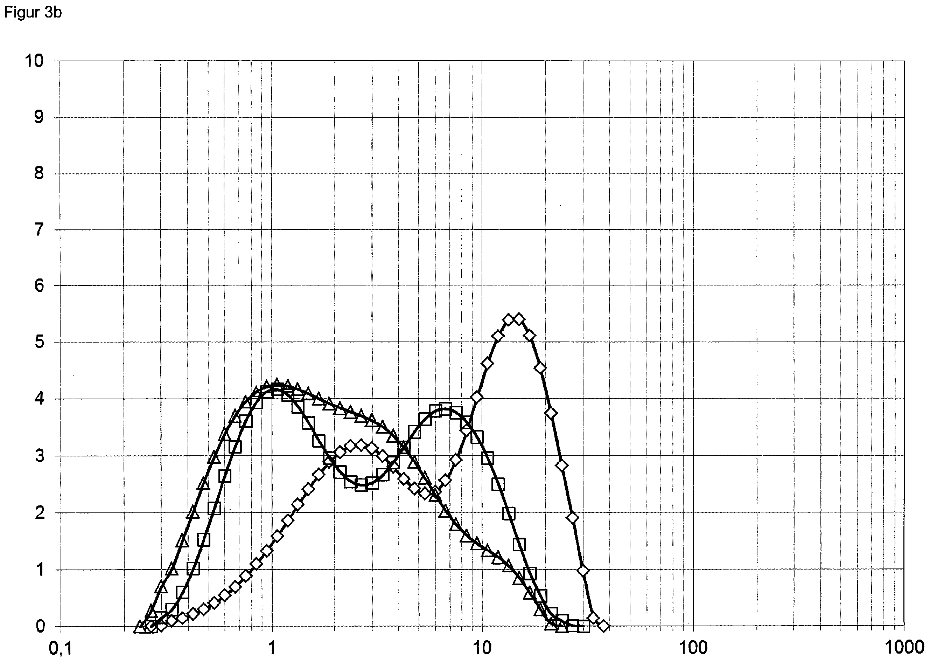

Die Größenverteilung der Partikel des vorstehenden gemahlenen katalytisch aktiven Multielementoxidmassenpulvers zeigen die Figuren 3a und 3b dieser Anmeldung (dieser Schrift) in Abhängigkeit vom Dispergierdruck der zur Trockendispergierung verwendeten Druckluft ($\diamond$ = 1,1 bar abs.; $\square$ = 2,0 bar abs.; $\Delta$ = 4,5 bar abs.). In der Figur 3a zeigt die Abszisse in logarithmischer Auftragung (im logarithmischen Maßstab zur Basis 10)) den Partikeldurchmesser (die Partikelausdehnung) in $\mu$m und der zu einem bestimmten Partikeldurchmesser auf der Abszisse gehörige Ordinatenwert auf der Verteilungskurve zeigt die X-% des Gesamtpartikelvolumens, die aus Partikeln mit dieser Partikelausdehnung bestehen. In der Figur 3b zeigt die Abszisse wieder in logarithmischer Auftragung (im logarithmischen Maßstab zur Basis 10) den Partikeldurchmesser (die Partikelausdehnung) in $\mu$m. Die Ordinate zeigt hier jedoch den Volumenanteil des Gesamtpartikelvolumens, der den jeweiligen Durchmesser oder einen kleineren Durchmesser aufweist.

Die der Partikeldurchmesserverteilung der Figuren 3a und 3b zugrunde liegende Messmethode ist die in dieser Schrift bereits ausgeführte Laserbeugung. Dabei wurde das Multielementoxidmassenpulver über eine Dispergierrinne in den Trockendispergierer Sympatec RODOS (Sympatec GmbH, System-Partikel-Technik, Am Pulverhaus 1, D-38678 Clausthal-Zellerfeld) geführt, dort mit Druckluft (die den jeweiligen Dispergierdruck von 1,1 bzw. 2 bzw. 4,5 bar abs. aufwies) trocken dispergiert und im Freistrahl an die Messzelle geblasen. In dieser wurde dann nach ISO 13320 mit dem Laserbeugungsspektrometer Malvern Mastersizer S (Malvern Instruments, Worcestershire WR14 1AT, United Kingdom) die volumenbezogene Partikeldurchmesserverteilung bestimmt (obscuration 3-7 %).

1,74 kg des gemahlenen $Mo_{12}V_3W_{1,2}Cu_{1,2}O_x$ Pulvers, das die in den Figuren 3a und 3b dieser Schrift in Abhängigkeit vom angewandten Dispergierdruck gezeigten Partikelgrößenverteilungen aufwies, wurde mit 0,261 kg (= 15 Gew.-% bezogen auf die 1,74 kg an Multielementoxidmasse) an feinteiligem $MoO_3$ versetzt (es handelte sich um "pure Molybdenum Oxide (POC)" der Climax Molybdenum Marketing Corporation, Phoenix (Arizona), USA, das zuvor noch in einer Spiralmühle (Eigenbau der Fa. BASF SE mit einem Mahlraumdurchmesser von 450 mm) gemahlen worden war, und nach der Mahlung in Abhängigkeit vom jeweils angewandten Dispergierdruck die Partikeldurchmesserverteilungen gemäß der Figuren 2a und 2b dieser Schrift aufwies ($\diamond$ = 1,1 bar abs.; $\square$ = 2,0 bar abs.; $\Delta$ = 4,5 bar abs.)).

Die nachfolgende Tabelle 1 zeigt für das feinteilige $MoO_3$ in Abhängigkeit vom jeweils angewandten Dispergierdruck die jeweiligen Werte für $d_{10}$, $d_{50}$ und $d_{90}$ (in $\mu$m) vor und nach der Mahlung.

Tabelle 1

| MoO$_3$ | Dispergierdruck [bar abs.] | $d_{10}$ [um] | $d_{50}$ [$\mu$m] | $d_{90}$ [$\mu$m] |
|---|---|---|---|---|
| vor Mahlung | 1,1 | 87,7 | 233,95 | 440,18 |
| | 2,0 | 31,27 | 186,93 | 381,42 |
| | 4,5 | 0,71 | 90,13 | 292,86 |
| nach Mahlung | 1,1 | 18,52 | 41,12 | 167,14 |
| | 2,0 | 0,39 | 0,78 | 1,89 |
| | 4,5 | 0,35 | 0,68 | 1,33 |

Die spezifische BET-Oberfläche des MoO$_3$ vor der Mahlung war 3 + 1 m$^2$/g.
Die spezifische BET-Oberfläche des MoO$_3$ nach der Mahlung war 5 + 1 m$^2$/g.
Die Zugabe des MoO$_3$ erfolgte portionsweise (fünf gleichgroße Portionen) innerhalb von 5 Minuten in das in einem Eirich-Intensivmischer vom Typ RV 02 (der Fa. Gustav Eirich GmbH & Co. KG in D-74736 Hardheim) vorgelegte gemahlene Mo$_{12}$V$_3$W$_{1,2}$Cu$_{1,2}$O$_x$ Pulver hinein. Die Wirbler und Teller des Eirichmischers wurden bei jeder MoO$_3$-Zugabe aus- und dann wieder eingeschaltet (Einstellungen: Teller: 1. Stufe; Wirbler: 2. Stufe). Nachdem das MoO$_3$ Pulver vollständig hinzugegeben worden war, wurde das im Eirich-Intensivmischer befindliche Gemisch über einen weiteren Zeitraum von 2 Minuten noch zusätzlich homogen durchmischt.
Mit dem so erzeugten feinteiligen Gemisch (seine spezifische innere Gesamtporenoberfläche betrug 8,6 m$^2$/g; sein spezifisches inneres Porengesamtvolumen (das Gesamtintrusionsvolumen) war 0,42 ml/g) wurde, wie im Vergleichsbeispiel 1A der WO 2011/134932 A1 auf Seite 8 ff. beschrieben, ein ringförmiger Vergleichskatalysator VS hergestellt. Die dem Umlufttrockenschrank (Typ UM 400 der Firma Memmert GmbH + Co. KG in DE 91126 Schwabach; Innenvolumen = 53 l; Luftstrom = 800 Nl/h; die Temperatur im Umlufttrockenschrank (einschließlich der Lufttemperatur) war während des Entfeuchtens 300 °C; das Trockengut befand sich während der Trocknung auf einem im Trockenschrank zentriert platzierten Lochblech (der Lochdurchmesser der über das Lochblech gleichmäßig verteilten Durchtrittsöffnungen = 0,5 cm; das Öffnungsverhältnis des Lochblechs war 60%; die Gesamtquerschnittsfläche des Lochblechs war 35 cm x 26 cm = 910 cm$^2$) geschichtet (Schichthöhe = 2 cm)) entnommenen ringförmigen Vergleichsschalenkatalysatoren VS wiesen, bezogen auf ihre Gesamtmasse, einen oxidischen Schalenanteil von 22,7 Gew.-% auf. Die spezifische BET-Oberfläche der Vergleichsschalenkatalysatoren VS betrug 3,15 m$^2$/g. Als geometrische Trägerformkörper wurden Ringe (7 mm Außendurchmesser, 3 mm Länge und 4 mm Innendurchmesser) aus Steatit C220 mit einer Oberflächenrauigkeit Rz von 45 $\mu$m (Splittauflage) der Fa. CeramTec verwendet. Das auf das Volumen der Trägerformkörpermasse bezogene Porengesamtvolumen des Trägerkörpers betrug $\leq$ 1 Vol.-%. Die spezifische BET-Oberfläche der geometrischen Trägerformkörper betrug 0,04 m$^2$/g.
Damit errechnet sich die spezifische BET-Oberfläche X der Aktivmassenschale der Vergleichsschalenkatalysatoren VS aus

$$0,227 \text{ m}^2/\text{g} \times X \text{ m}^2/\text{g} + 0,773 \times 0,04 \text{ m}^2/\text{g} = 3,15 \text{ m}^2/\text{g}$$

zu X = 13,7 m$^2$/g.
Untersuchungen der Porenbeschaffenheit der Aktivmassenschale der Vergleichsschalenkatalysatoren VS ergaben folgende Ergebnisse:
Die spezifische innere Gesamtporenoberfläche der untersuchten Poren der Aktivmassenschale der Vergleichsschalenkatalysatoren VS betrug 6,71 m$^2$/g.
Das zugehörige spezifische Porengesamtvolumen war 0,3 ml/g.

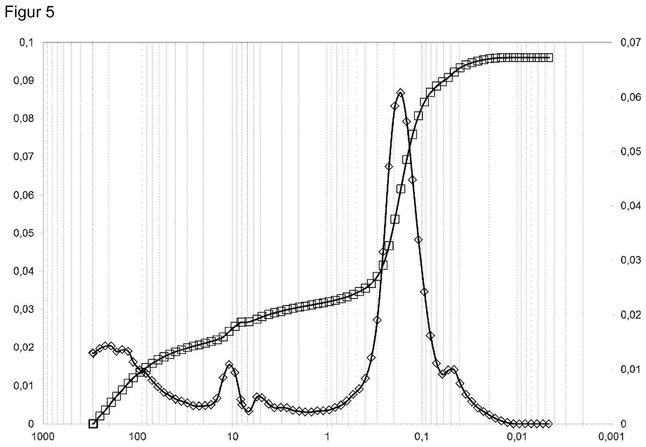

Figur 5 der vorliegenden Schrift zeigt die Porendurchmesserverteilung der Poren der Aktivmassenschale der Vergleichskatalysatoren VS. Auf der Abszisse ist der jeweilige Porendurchmesser in $\mu$m aufgetragen (logarithmische Auftragung zur Basis 10). Auf der linken Ordinate ist der Logarithmus (zur Basis 10) des differentiellen Beitrags in ([ml]/[g Schalenkatalysator]) des jeweiligen Porendurchmessers zum spezifischen (hier auf die Gesamtmasse gebildet aus der Masse des geometrischen Trägerformkörpers und der Masse der Aktivmassenschale bezogenen) Porengesamtvolumen aufgetragen (Kurve $\diamondsuit$). Das Maximum weist den Porendurchmesser mit dem größten Beitrag zum (spezifischen) Porengesamtvolumen aus. Auf der rechten Ordinate ist in ([ml]/[g Schalenkatalysator]) das Integral über die individuellen Beiträge der einzelnen Porendurchmesser zum vorgenannten spezifischen Porengesamtvolumen (der kumulative Beitrag zum vorgenannten spezifischen Porengesamtvolumen) aufgetragen (Kurve □). Der Endpunkt ist das auf die Gesamtmasse von geometrischem Trägerformkörper und Aktivmasse bezogene (spezifische) Porengesamtvolumen (Gesamtintrusionsvolumen). Durch Division dieses Wertes mit 0,227 resultiert das auf die Aktivmasse bezogene spezifische Porengesamtvolumen.

Angaben zur Porenbeschaffenheit von festen Stoffen beziehen sich in der vorliegenden Schrift (soweit nicht ausdrücklich etwas anderes gesagt wird) stets auf eine Analyse des jeweiligen Festkörpers mit der Methode der Quecksilber-Porosimetrie. Diese Methode verwendet die die meisten Stoffe nicht benetzende Flüssigkeit Quecksilber, um Informationen über die Porenbeschaffenheit des untersuchten porösen Festkörpers zu erhalten.

Dabei wird das zuvor ausgegaste (um in der porösen Struktur gegebenenfalls enthaltene Flüssigkeit auszugasen) poröse System (die zu untersuchende Probe) in ein Quecksilberbad getaucht, dessen Druck verändert werden kann.

Da das Quecksilber das Probenmaterial nicht benetzt, muss das Quecksilber in die Poren der Probe gedrückt werden (beim jeweiligen Druck wird Gleichgewichtseinstellung abgewartet). Das Eindringen des Quecksilbers in Poren mit größerer Querschnittsfläche erfolgt bei vergleichsweise geringeren Drucken, während das Eindringen des Quecksilbers in engere Poren einen vergleichsweise höheren Druck erfordert. Unter der Annahme des Vorliegens von kreiszylindrischen Poren kann mit Hilfe der Washburn-Gleichung der äußere Druck, der erforderlich ist, um das flüssige Quecksilber gegen die Oberflächenspannung des Quecksilbers in die Poren eines entsprechenden Durchmessers zu drücken (zu intrudieren; Quecksilberintrusion), zum besagten Durchmesser in Beziehung gesetzt werden. Der im Rahmen der Quecksilber-Porosimetrie-Untersuchung angewandte Druckbereich korreliert mit der (Band)Breite der erfassten Porendurchmesser.

Aus den bei 25 °C experimentell ermittelten Quecksilberintrusionskurven kann anschließend über die erfasste (Band)Breite der Porendurchmesser die Durchmesserverteilung der Poren, die innere Gesamtoberfläche der Poren und das innere Gesamtvolumen der Poren (das Gesamtintrusionsvolumen; das Porengesamtvolumen) rechnerisch extrahiert werden (vgl. Inauguraldissertation "Eigenschaften und Einsatzmöglichkeiten von Aerogelfenstern im Vergleich mit konventionellen sowie evakuierten Fenstern" von Georges Reber (1991), an der Philosophischnaturwissenschaftlichen Fakultät der Universität Basel). Das nachfolgend beschriebene Messgerät Auto Pore IV 9520 der Firma Micromeritics umfasst diesbezüglich geeignete Standardrechenprogramme.

In dieser Schrift beziehen sich alle Angaben zur Porenbeschaffenheit von festen Stoffen, soweit nichts anderes explizit erwähnt wird, auf Bestimmungen mit der Methode der Quecksilberporosimetrie in Anwendung eines Gerätes Auto Pore IV 9520 der Firma Micromeritics in Norcross, Georgia 30093-1877, USA. Im Fall von untersuchten Pulvern betrug die in den Probenraum jeweils eingebrachte Probenmenge 2,5 g. Im Fall von untersuchten Schalenkatalysatoren wurden jeweils 5 Stücke des jeweiligen Schalenkatalysators in den Probenraum eingebracht (der Beitrag der Poren des geometrischen Trägerformkörpers des Schalenkatalysators war dabei in den untersuchten Fällen im Vergleich zum Beitrag der Poren der Aktivmassenschale vernachlässigbar).

Der Probenraum war in eine langgezogene Kapillare fortgeführt, sodass geringen Druckänderungen deutliche Änderungen der Länge des in die Kapillare ragenden Quecksilberfadens entsprachen. Das genutzte Kapillarvolumen lag in allen Fällen zwischen 25 und 91 Vol.-%, bezogen auf das Kapillargesamtvolumen.

Vor Beginn einer jeweiligen Probenuntersuchung wurde der Probenraum (bei 25 °C) jeweils bis zu einem Innendruck von $9,3 \times 10^{-4}$ bar evakuiert und die Probe während 20 Minuten bei dieser Temperatur und bei diesem Druck entgast. Danach wurde das Quecksilber mit über die Zeit ansteigenden Drucken bis zu einem Enddruck von 4137 bar in den Probenraum gedrückt. Der Anfangsdruck betrug 0,04 bar. Dem entspricht eine (Band)Breite an erfassten Porendurchmessern von 0,003 μm bis 360 μm.

B) Herstellung eines ringförmigen erfindungsgemäßen Schalenkatalysators ES

Wie für die Herstellung des Multielementoxids der Stöchiometrie $Mo_{12}V_3W_{1,2}Cu_{1,2}O_n$ im Ausführungsbeispiel 1A auf Seite 9 der WO 2011/134932 A1 beschrieben, wurde ein dem entsprechendes Sprühpulver erzeugt. Die Größenverteilung der Partikel des Sprühpulvers zeigen die Figuren 4a und 4b dieser Anmeldung (der vorliegenden Schrift) in Abhängigkeit vom Dispergierdruck der zur Trockendispergierung verwendeten Druckluft (◇ = 1,1 bar abs.; □ = 2,0 bar abs.; Δ = 4,5 bar abs.).

In der Figur 4a zeigt die Abszisse in logarithmischer Auftragung (im logarithmischen Maßstab zur Basis 10) den Partikeldurchmesser (die Partikelausdehnung) in μm und der zu einem bestimmten Partikeldurchmesser auf der Abszisse gehörige Ordinatenwert auf der Verteilungskurve zeigt die X-% des Gesamtpartikelvolumens, die aus Partikeln mit dieser Partikelausdehnung bestehen. In der Figur 4b zeigt die Abszisse wieder in logarithmischer Auftragung (im logarithmischen Maßstab zur Basis 10) den Partikeldurchmesser (die Partikelausdehnung) in μm. Die Ordinate zeigt hier jedoch den Volumenanteil des Gesamtpartikelvolumens, der den jeweiligen Durchmesser oder einen kleineren Durchmesser aufweist. Die der Partikeldurchmesserverteilung der Figuren 4a und 4b zugrunde liegende Messmethode ist die in dieser Schrift bereits mehrfach ausgeführte Laserbeugung.

Die nachfolgende Tabelle 2 zeigt für das Sprühpulver in Abhängigkeit vom jeweils angewandten Dispergierdruck die Werte für $d_{10}$, $d_{50}$ und $d_{90}$ (in $\mu$m).

Tabelle 2

| Dispergierdruck [bar abs.] | $d_{10}$ [um] | $d_{50}$ [um] | $d_{90}$ [um] |
|---|---|---|---|
| 1,1 | 5,89 | 22,66 | 64,43 |
| 2,0 | 2,89 | 12,62 | 53,24 |
| 4,5 | 1,58 | 7,89 | 40,74 |

Zu 1000 g des resultierenden und auf 25 °C abgekühlten Sprühpulvers (dessen (Rest)Wassergehalt 4,2 Gew.-% betrug), die in einem Kneter der Fa. Werner & Pfleiderer vom Typ ZS1-80 vorgelegt waren, wurden 150 g des bei der Herstellung des Vergleichsschalenkatalysators VS (siehe A) dieser Schrift) gemahlenen $MoO_3$ hinzugefügt. Unter Zusatz von 170 g Wasser und 200 g einer 50 gew.-%igen wässrigen Essigsäurelösung, die beide eine Temperatur von 25 °C aufwiesen, wurde das Feststoffgemisch im Kneter verknetet (Knetdauer: 2 Stunden; Knettemperatur: 30 bis 35 °C).

Anschließend wurde das Knetgut zu kreiszylindrischen Stränglingen (Länge: 20 bis 30 cm, Durchmesser: 6 mm) extrudiert (grundsätzlich können die vorgenannte Knetung und das Extrudieren auch in einem einzigen Apparat, einem sogenannten Auspresskneter (der Fa. AMK (Aachener Misch- u. Knetmaschinen - Fabrik Peter Küpper GmbH & Co. KG, D-52074 Aachen) vom Typ: VI U 2.5/IV) durchgeführt werden; großtechnisch würde an dieser Stelle ein Auspresskneter der Firma AMK vom Typ VI U - 160 (Maschinen Nr. C12566) eingesetzt werden) und diese in einer Schichtdicke von 2 cm (auf ein im Trockenschrank zentriert platziertes Lochblech (der Lochdurchmesser der über das Lochblech gleichmäßig verteilten Durchtrittsöffnungen = 0,5 cm; das Öffnungsverhältnis des Lochblechs war 60%; die Gesamtquerschnittsfläche des Lochblechs war 35 cm x 26 cm = 910 cm²) geschichtet) während 16 h in einem Umlufttrockenschrank (Typ UT 6 der Firma Heareus) getrocknet (Innenvolumen = 57 l; Luftstrom = 2850 l/h; die Temperatur im Trockenschrank (einschließlich der Lufttemperatur) war 110 °C). Der Restwassergehalt der getrockneten Stränglinge betrug (bezogen auf ihr Gewicht) 1,85 Gew.-%; der Glühgewichtsverlust der getrockneten Stränglinge war (bezogen auf ihr Gewicht) 20,56 Gew.%.

Die thermische Weiterbehandlung der getrockneten geometrischen Vorläuferformkörper erfolgte in entsprechender Weise wie im Vergleichsbeispiel 1A der WO 2011/134932 A1 auf den Seiten 7 und 8 ausgeführt.

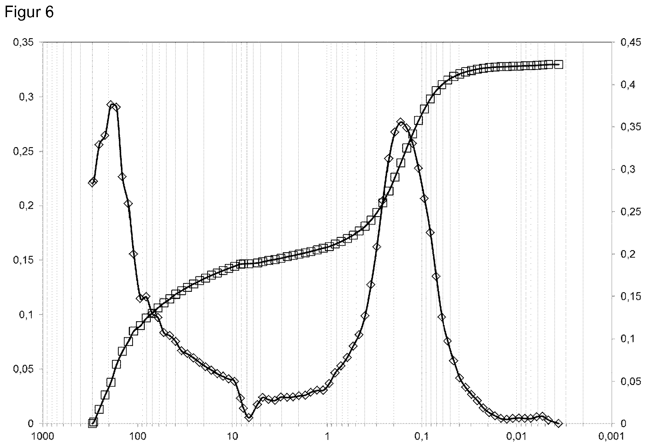

Die dem Drehrohrofen entnommenen geometrischen Katalysatorformkörper wurden anschließend wie auf Seite 8 der WO 2011/134932 A1 beschrieben zu einem feinteiligen (Aktivmassen)Pulver gemahlen (die spezifische BET-Oberfläche des Aktivmassenpulvers war 17 m²/g; die spezifische innere Gesamtporenoberfläche des Aktivmassenpulvers betrug 8,65 m²/g; das spezifische innere Porengesamtvolumen des Aktivmassenpulvers (das Gesamtintrusionsvolumen) war 0,42 ml/g; Figur 6 der vorliegenden Schrift zeigt die Porendurchmesserverteilung der Poren des Aktivmassenpulvers; auf der Abszisse ist der jeweilige Porendurchmesser in $\mu$m aufgetragen (logarithmische Auftragung zur Basis 10); auf der linken Ordinate ist der Logarithmus (zur Basis 10) des differentiellen Beitrags in ([ml]/[g Aktivmassenpulver]) des jeweiligen Porendurchmessers zum spezifischen (hier auf die Masse des Aktivmassenpulvers bezogenen) Porengesamtvolumen aufgetragen (Kurve $\diamond$); das Maximum weist den Porendurchmesser mit dem größten Beitrag zum (spezifischen) Porengesamtvolumen aus; auf der rechten Ordinate ist in ([ml]/[g Aktivmassenpulver]) das Integral über die individuellen Beiträge der einzelnen Porendurchmesser zum vorgenannten spezifischen Porengesamtvolumen (der kumulative Beitrag zum vorgenannten spezifischen Porengesamtvolumen) aufgetragen (Kurve $\square$); der Endpunkt ist das auf die Masse des Aktivmassenpulvers bezogene (spezifische) Porengesamtvolumen (Gesamtintrusionsvolumen)) und selbiges wie auf den Seiten 8/9 der WO 2011/134932 A1 beschrieben, zur Herstellung eines erfindungsgemäßen ringförmigen Schalenkatalysators ES verwendet (gleicher geometrische Trägerformkörper wie bei der Herstellung des ringförmigen Vergleichsschalenkatalysators VS unter A) dieser Schrift).

Die dem Umlufttrockenschrank (Typ UM 400 der Firma Memmert GmbH + Co. KG in DE 91126 Schwabach; Innenvolumen = 53 l; Luftstrom = 800 Nl/h; die Temperatur im Umlufttrockenschrank (einschließlich der Lufttemperatur) war während des Entfeuchtens 300 °C; das Trockengut befand sich während der Trocknung auf einem im Trockenschrank zentriert platzierten Lochblech (der Lochdurchmesser der über das Lochblech gleichmäßig verteilten Durchtrittsöffnungen = 0,5 cm; das Öffnungsverhältnis des Lochblechs war 60%; die Gesamtquerschnittsfläche des Lochblechs war 35 cm x 26 cm = 910 cm²) geschichtet (Schichthöhe = 2 cm)) entnommenen erfindungsgemäßen ringförmigen Schalenkatalysatoren ES wiesen, bezogen auf ihre Gesamtmasse, einen oxidischen Schalenanteil von 22,8 Gew.-% auf. Die spezifische BET-Oberfläche der Schalenkatalysatoren ES betrug 3,9 m²/g.

Damit errechnet sich die spezifische BET-Oberfläche Y der Aktivmassenschale der erfindungsgemäßen Schalenkatalysatoren ES aus

$$0,228 \text{ m}^2/\text{g x Y m}^2/\text{g} + 0,772 \times 0,04 \text{ m}^2/\text{g} = 3,9 \text{ m}^2/\text{g}$$

zu Y = 17 m$^2$/g.

Untersuchungen der Porenbeschaffenheit der Aktivmassenschale der erfindungsgemäßen Schalenkatalysatoren ES ergaben folgende Ergebnisse:
Die spezifische innere Gesamtporenoberfläche der untersuchten Poren der Aktivmassenschale der erfindungsgemäßen Schalenkatalysatoren ES betrug 7,41 m$^2$/g.

Das zugehörige spezifische Porengesamtvolumen war 0,27 ml/g.

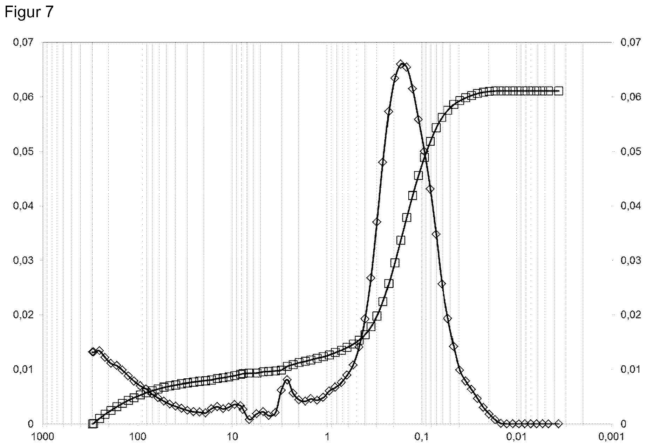

Figur 7 der vorliegenden Schrift zeigt die Porendurchmesserverteilung der Poren der Aktivmassenschale der erfindungsgemäßen Schalenkatalysatoren ES. Auf der Abszisse ist der jeweilige Porendurchmesser in μm aufgetragen (logarithmische Auftragung zur Basis 10). Auf der linken Ordinate ist der Logarithmus (zur Basis 10) des differentiellen Beitrags in ([ml]/[g Schalenkatalysator]) des jeweiligen Porendurchmessers zum spezifischen (hier auf die Gesamtmasse gebildet aus der Masse des geometrischen Trägerformkörpers und der Masse der Aktivmassenschale bezogenen) Porengesamtvolumen aufgetragen (Kurve ◇). Das Maximum weist den Porendurchmesser mit dem größten Beitrag zum (spezifischen) Porengesamtvolumen aus. Auf der rechten Ordinate ist in ([ml]/[g Schalenkatalysator]) das Integral über die individuellen Beiträge der einzelnen Porendurchmesser zum vorgenannten spezifischen Porengesamtvolumen (der kumulative Beitrag zum vorgenannten spezifischen Porengesamtvolumen) aufgetragen (Kurve □). Der Endpunkt ist das auf die Gesamtmasse von geometrischem Trägerformkörper und Aktivmasse bezogene (spezifische) Porengesamtvolumen (Gesamtintrusionsvolumen). Durch Division dieses Wertes mit 0,228 resultiert das auf die Aktivmasse bezogene spezifische Porengesamtvolumen.

Wurde zur Schalenkatalysatorherstellung ausschließlich das in den Figuren 3a und 3b der vorliegenden Schrift mit seiner Partikelgrössenverteilung gezeigte gemahlene Aktivmassenpulver der Stöchiometrie Mo$_{12}$V$_3$W$_{1,2}$Cu$_{1,2}$O$_x$ verwendet und im Übrigen wie in A) der vorliegenden Schrift zur Herstellung des Vergleichsschalenkatalysators VS beschrieben verfahren, resultierten ringförmige Vergleichsschalenkatalysatoren mit einem oxidischen Aktivmassenanteil von 22,3 Gew.-% (diese können, in entsprechender Weise wie in dieser Schrift für erfindungsgemäße Schalenkatalysatoren ES beschrieben, auch zur Katalyse der heterogen katalysierten Partialoxidation von Acrolein zu Acrylsäure eingesetzt werden). Untersuchungen der Porenbeschaffenheit der Aktivmassenschale dieser Vergleichsschalenkatalysatoren ergaben folgende Ergebnisse:
Die spezifische innere Gesamtporenoberfläche der untersuchten Poren der Aktivmassenschale dieser Vergleichsschalenkatalysatoren betrug 5,88 m$^2$/g.

Das zugehörige spezifische Porengesamtvolumen war 0,20 ml/g.

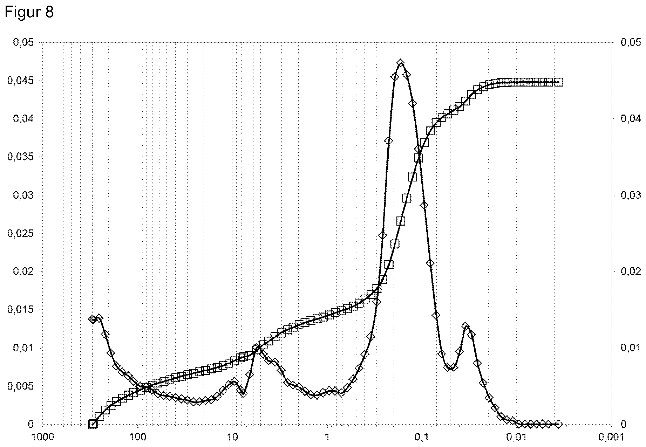

Figur 8 der vorliegenden Schrift zeigt die Porendurchmesserverteilung der Poren der Aktivmassenschale dieser Vergleichsschalenkatalysatoren. Auf der Abszisse ist der jeweilige Porendurchmesser in μm aufgetragen (logarithmische Auftragung zur Basis 10). Auf der linken Ordinate ist der Logarithmus (zur Basis 10) des differentiellen Beitrags in ([ml]/[g Schalenkatalysator]) des jeweiligen Porendurchmessers zum spezifischen (hier auf die Gesamtmasse gebildet aus der Masse des geometrischen Trägerformkörpers und der Masse der Aktivmassenschale bezogenen) Porengesamtvolumen aufgetragen (Kurve ◇). Das Maximum weist den Porendurchmesser mit dem größten Beitrag zum (spezifischen) Porengesamtvolumen aus. Auf der rechten Ordinate ist in ([ml]/[g Schalenkatalysator]) das Integral über die individuellen Beiträge der einzelnen Porendurchmesser zum vorgenannten spezifischen Porengesamtvolumen (der kumulative Beitrag zum vorgenannten spezifischen Porengesamtvolumen) aufgetragen (Kurve □). Der Endpunkt ist das auf die Gesamtmasse von geometrischem Trägerformkörper und Aktivmasse bezogene (spezifische) Porengesamtvolumen (Gesamtintrusionsvolumen). Durch Division dieses Wertes mit 0,223 resultiert das auf die Aktivmasse bezogene spezifische Porengesamtvolumen.

C) Testung der Schalenkatalysatoren VS sowie ES als Katalysatoren für die heterogen katalysierte partielle Gasphasenoxidation von Acrolein zu Acrylsäure

Ein Reaktionsrohr (V2A Stahl; 30 mm Außendurchmesser; 2 mm Wandstärke; 26 mm Innendurchmesser; 464 cm Länge) wurde von oben nach unten wie folgt beschickt:

Abschnitt 1:     79 cm Länge Leerrohr;
Abschnitt 2:     62 cm Länge Vorschüttung aus Steatit-Ringen der Geometrie 7 mm x 3 mm x 4 mm
                 (Außendurchmesser x Länge x Innendurchmesser; Steatit C220 der Fa. CeramTec);

(fortgesetzt)

| | | |
|---|---|---|
| Abschnitt 3: | 100 cm Länge Katalysatorfestbettschüttung aus einem homogenen Gemisch bestehend aus 20 Gew.-% Steatit-Ringen der Geometrie 7 mm x 3 mm x 4 mm (Außendurchmesser x Länge x Innendurchmesser); Steatit C220 der Fa. CeramTec) und 80 Gew.-% des jeweiligen Schalenkatalysators; |
| Abschnitt 4: | 200 cm Länge Katalysatorfestbettschüttung ausschließlich bestehend aus dem in Abschnitt 3 jeweils verwendeten Schalenkatalysator; |
| Abschnitt 5: | 10 cm Länge Nachschüttung aus denselben Steatit-Ringen wie in Abschnitt 2; |
| Abschnitt 6: | 14 cm Länge Katalysatorstuhl aus V2A Stahl zur Aufnahme des Katalysatorfestbetts |

Durch das jeweilige wie vorstehend beschrieben beschickte Reaktionsrohr wurde von oben nach unten durch das Reaktionsrohr strömend ein Reaktionsgasgemisch geführt, das folgende Gehalte aufwies:

| | | |
|---|---|---|
| 4,3 | Vol.-% | Acrolein, |
| 0,2 | Vol.-% | Propen, |
| 0,2 | Vol.-% | Propan, |
| 0,3 | Vol.-% | Acrylsäure, |
| 5,4 | Vol.-% | $O_2$, |
| 7 | Vol.-% | $H_2O$, |
| 0,4 | Vol.-% | CO und $CO_2$, und |
| 82,2 | Vol.-% | $N_2$. |

Die Belastung des Katalysatorfestbetts (wie in der vorliegenden Schrift definiert) mit Acrolein betrug jeweils 75 Nl/l•h. Das Reaktionsgasgemisch wurde dem Reaktionsrohr mit einer Temperatur von 255 °C zugeführt. Der Arbeitsdruck am Eingang in das Reaktionsrohr war 1,6 bar (abs.).

Das Reaktionsrohr war über seine Länge (bis auf die letzten 10 cm des Leerrohres im Abschnitt 1 und die letzten 3 cm des Rohres im Abschnitt 6) jeweils von einem gerührten und von außen elektrisch beheizten Salzbad (Gemisch aus 53 Gew.-% Kaliumnitrat, 40 Gew.-% Natriumnitrit und 7 Gew.-% Natriumnitrat; 50 kg Salzschmelze) umspült (die Strömungsgeschwindigkeit am Rohr war 3 m/s (in der Ebene senkrecht zur Längsachse des Rohres)).

Über die Rohrlänge betrachtet strömte das Salzbad entgegengesetzt zum Reaktionsgasgemisch mit einer Strömungsgeschwindigkeit von 3 m/s.

Die Salzbadtemperatur $T^B$ (°C) (mit der das Salzbad zugeführt wurde) wurde in allen Fällen so eingestellt, dass ein auf den einfachen Durchgang des Reaktionsgasgemischs durch das Katalysatorfestbett bezogener Acroleinumsatz U von 99,3 mol-% resultierte. Entlang des Reaktionsrohres änderte sich die Salzbadtemperatur infolge Zuheizung nicht (es wurde vom Salzbad mehr Wärme abgestrahlt wie vom Reaktionsrohr an das Salzbad abgegeben). Die Zuführtemperatur des Reaktionsgasgemischs (am Eingang in das Reaktionsrohr) war jeweils auf die jeweilige Salzbadtemperatur eingestellt.

Die nachfolgende Tabelle 3 zeigt die in Abhängigkeit vom eingesetzten Schalenkatalysator nach jeweils 100 Betriebsstunden resultierenden Ergebnisse, wobei S die Selektivität der Acrylsäurebildung ist.

Tabelle 3

| Schalenkatalysator | $T^B$ [°C] | U [mol-%] | S [mol-%] |
|---|---|---|---|
| VS | 261 | 99,3 | 96,7 |
| ES | 248 | 99,3 | 97,3 |

Sowohl bei Verwendung des Schalenkatalysators VS als auch bei Verwendung des Schalenkatalysators ES war über eine Betriebsdauer von 55 Tagen keine Erhöhung der Salzbadtemperatur zum Beibehalt des Zielumsatzes erforderlich. Die Selektivität der Acrylsäurebildung war in beiden Fällen über die gesamte Betriebsdauer stabil.

Die in der Tabelle gezeigten Ergebnisse weisen aus, dass bei erfindungsgemäßer Einarbeitung des $MoO_3$ in die katalytisch aktive Masse nicht nur ein voll befriedigendes Langzeitverhalten derselben sondern zusätzlich sowohl eine höhere Aktivität als auch eine höhere Selektivität der Zielproduktbildung resultiert.

Sowohl die Schalenkatalysatoren VS als auch die Schalenkatalysatoren ES konnten bei Temperaturen von 20 bis 40 °C unter Umgebungsluft mit bis zu 90 % oder mehr relativer Luftfeuchtigkeit gelagert werden, ohne an katalytischer Aktivität zu verlieren.

US Provisional Patent Application No. 61/761,812, eingereicht am 7. Februar 2013, ist in die vorliegende Anmeldung durch Literaturhinweis eingefügt.

Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

**Patentansprüche**

1. Verfahren zur Herstellung einer katalytisch aktiven Masse, die ein Gemisch aus einem die Elemente Mo und V enthaltenden Multielementoxid und wenigstens einem Oxid des Molybdäns ist, **dadurch gekennzeichnet, dass** das molare Verhältnis Mo /V, der im Multielementoxid enthaltenen molaren Mengen an Mo und an V, 15 : 1 bis 1 : 1 beträgt und als Verfahrensmaßnahmen

   - mit Quellen der elementaren Konstituenten des Multielementoxids eine wässrige Lösung oder mit der Maßgabe eine wässrige Suspension erzeugt wird, dass jede der Quellen beim Erzeugen der wässrigen Suspension den Zustand einer wässrigen Lösung durchläuft,
   - durch Sprühtrocknung der wässrigen Lösung oder der wässrigen Suspension ein Sprühpulver P erzeugt wird,
   - mit dem Sprühpulver P unter Zusatz wenigstens eines pulverförmigen Oxids S des Molybdäns sowie wahlweise unter Zusatz eines oder mehrerer Formgebungshilfsmittel und nach gleichmäßiger Vermischung der genannten Bestandteile aus dem dabei resultierenden Gemisch geometrische Vorläuferformkörper geformt, wobei das wenigstens eine pulverförmige Oxid S des Molybdäns Molybdändioxid, Molybdäntrioxid oder ein Gemisch aus Molybdändioxid und Molybdäntrioxid ist, und
   - die geometrischen Vorläuferformkörper unter Ausbildung der katalytisch aktiven Masse thermisch behandelt werden.

2. Verfahren nach Anspruch 1, **dadurch gekennzeichnet, dass** die spezifische Oberfläche $O_M$ des wenigstens einen pulverförmigen Oxids S des Molybdäns $\leq 20$ m$^2$/g beträgt.

3. Verfahren nach einem der Ansprüche 1 oder 2, **dadurch gekennzeichnet, dass** die spezifische Oberfläche $O_M$ des wenigstens einen pulverförmigen Oxids S des Molybdäns $\geq 0{,}01$ m$^2$/g beträgt.

4. Verfahren nach einem der Ansprüche 1 bis 3, **dadurch gekennzeichnet, dass** der Partikeldurchmesser $d_{90}$ nach ISO 13320 des wenigstens einen pulverförmigen Oxids S des Molybdäns $\leq 20$ $\mu$m beträgt.

5. Verfahren nach einem der Ansprüche 1 bis 4, **dadurch gekennzeichnet, dass** der Partikeldurchmesser $d_{10}$ nach ISO 13320 des wenigstens einen pulverförmigen Oxids S des Molybdäns $\geq 50$ nm beträgt.

6. Verfahren nach einem der Ansprüche 1 bis 5, **dadurch gekennzeichnet, dass** der Zusatz des wenigstens einen pulverförmigen Oxids S des Molybdäns, bezogen auf das Gewicht der beim Verfahren hergestellten katalytisch aktiven Masse, in einer Menge von > 0 und $\leq 50$ Gew.-% erfolgt.

7. Verfahren nach einem der Ansprüche 1 bis 6, **dadurch gekennzeichnet, dass** der molare Anteil des Elements Mo an der Gesamtmenge der von Sauerstoff verschiedenen Elemente des die Elemente Mo und V enthaltenden Multielementoxids 5 bis 95 mol-% beträgt.

8. Verfahren nach einem der Ansprüche 1 bis 7, **dadurch gekennzeichnet, dass** das Multielementoxid zusätzlich noch Cu enthält.

9. Verfahren nach einem der Ansprüche 1 bis 6, **dadurch gekennzeichnet, dass** die Stöchiometrie des Multielementoxids der nachfolgenden allgemeinen Stöchiometrie I,

$$Mo_{12}V_aX^1_bX^2_cX^3_dX^4_eX^5_fX^6_gO_n \qquad (I),$$

in der die Variablen folgende Bedeutung haben:

$X^1$ = W, Nb, Ta, Cr und/oder Ce,
$X^2$ = Cu, Ni, Co, Fe, Mn und/oder Zn,
$X^3$ = Sb und/oder Bi,
$X^4$ = eines oder mehrere Alkalimetalle (Li, Na, K, Rb, Cs) und/oder H,
$X^5$ = eines oder mehrere Erdalkallimetalle (Mg, Ca, Sr, Ba),
$X^6$ = Si, Al, Ti und/oder Zr,
a = 1 bis 6,
b = 0,2 bis 4,
c = 0 bis 18, vorzugsweise 0,5 bis 18,
d = 0 bis 40,
e = 0 bis 2,
f = 0 bis 4,
g = 0 bis 40, und
n = eine Zahl, die durch die Wertigkeit und Häufigkeit der von Sauerstoff verschiedenen Elemente in I bestimmt wird,

genügt.

10. Verfahren nach einem der Ansprüche 1 bis 6, **dadurch gekennzeichnet, dass** die Stöchiometrie des Multielementoxids der nachfolgenden allgemeinen Stöchiometrie II,

$$Mo_{12}V_a X^1_b X^2_c X^5_f X^6_g O_n \qquad (II),$$

in der die Variablen folgende Bedeutung haben:

$X^1$ = W und/oder Nb,
$X^2$ = Cu und/oder Ni,
$X^5$ = Co und/oder Sr,
$X^6$ = Si und/oder Al,
a = 3 bis 4,5,
b = 1 bis 1,5,
c = 0,75 bis 2,5,
f = 0 bis 0,5,
g = 0 bis 8, und
n = eine Zahl, die durch die Wertigkeit und Häufigkeit der von Sauerstoff verschiedenen Elemente in II bestimmt wird,

genügt.

11. Verfahren nach einem der Ansprüche 1 bis 10, **dadurch gekennzeichnet, dass** die thermische Behandlung der geometrischen Vorläuferformkörper eine Calcination bei 200 bis 600 °C umfasst.

12. Verfahren nach Anspruch 11, **dadurch gekennzeichnet, dass** sich die Calcination über einen Zeitraum von 0,5 h bis 24 h erstreckt.

13. Verfahren nach einem der Ansprüche 1 bis 12, **dadurch gekennzeichnet, dass** die katalytisch aktive Masse in eine feinteilige Form überführt und die feinteilige Form als eine Schale aus der katalytisch aktiven Masse auf die äußere Oberfläche eines geometrischen Trägerformkörpers aufgebracht wird.

14. Verfahren nach Anspruch 13, **dadurch gekennzeichnet, dass** die Dicke der aufgebrachten Schale aus katalytisch aktiver Masse 10 bis 1000 $\mu$m beträgt.

**Claims**

1. A process for producing a catalytically active composition being a mixture of a multielement oxide comprising the elements Mo and V and at least one oxide of molybdenum, wherein the molar Mo / V ratio of the molar amounts of Mo and V present in the multielement oxide is 15 : 1 to 1 : 1 and which process comprises the process measures of

- using sources of the elemental constituents of the multielement oxide to obtain an aqueous solution, or an aqueous suspension with the proviso that each of the sources passes through the state of an aqueous solution in the course of obtaining the aqueous suspension,
- spray drying the aqueous solution or aqueous suspension to obtain a spray powder P,
- using the spray powder P with addition of at least one pulverulent oxide S of molybdenum and optionally with addition of one or more shaping assistants and after homogeneous mixing of said constituents to form geometric shaped precursor bodies using the resulting mixture, where the at least one pulverulent oxide S of molybdenum is molybdenum dioxide, molybdenum trioxide or a mixture of molybdenum dioxide and molybdenum trioxide, and
- thermally treating the geometric shaped precursor bodies to form the catalytically active composition.

2. The process according to claim 1, wherein the specific surface area $O_M$ of the at least one pulverulent oxide S of molybdenum is $\leq 20$ $m^2/g$.

3. The process according to either of claims 1 and 2, wherein the specific surface area $O_M$ of the at least one pulverulent oxide S of molybdenum is $\geq 0.01$ $m^2/g$.

4. The process according to any of claims 1 to 3, wherein the particle diameter $d_{90}$ according to ISO 13320 of the at least one pulverulent oxide S of molybdenum is $\leq 20$ $\mu m$.

5. The process according to any of claims 1 to 4, wherein the particle diameter $d_{10}$ according to ISO 13320 of the at least one pulverulent oxide S of molybdenum is $\geq 50$ nm.

6. The process according to any of claims 1 to 5, wherein the at least one pulverulent oxide S of molybdenum, based on the weight of the catalytically active composition produced in the process, is added in an amount of > 0 and $\leq$ 50% by weight.

7. The process according to any of claims 1 to 6, wherein the molar proportion of the element Mo in the total amount of the elements other than oxygen in the multielement oxide comprising the elements Mo and V is 5 to 95 mol%.

8. The process according to any of claims 1 to 7, wherein the multielement oxide additionally comprises Cu.

9. The process according to any of claims 1 to 6, wherein the stoichiometry of the multielement oxide satisfies the following general stoichiometry I:

$$Mo_{12}V_a X^1_b X^2_c X^3_d X^4_e X^5_f X^6_g O_n \qquad (I)$$

in which the variables are each defined as follows:

$X^1$ = W, Nb, Ta, Cr and/or Ce,
$X^2$ = Cu, Ni, Co, Fe, Mn and/or Zn,
$X^3$ = Sb and/or Bi,
$X^4$ = one or more alkali metals (Li, Na, K, Rb, Cs) and/or H,
$X^5$ = one or more alkaline earth metals (Mg, Ca, Sr, Ba) ,
$X^6$ = Si, Al, Ti and/or Zr,
a = 1 to 6,
b = 0.2 to 4,
c = 0 to 18, preferably 0.5 to 18,
d = 0 to 40,
e = 0 to 2,
f = 0 to 4,
g = 0 to 40, and
n = a number which is determined by the valency and frequency of the elements in I other than oxygen.

10. The process according to any of claims 1 to 6, wherein the stoichiometry of the multielement oxide satisfies the following general stoichiometry II:

$$Mo_{12}V_a X^1_b X^2_c X^5_f X^6_g O_n \qquad (II)$$

in which the variables are each defined as follows:

$X^1$ = W and/or Nb,
$X^2$ = Cu and/or Ni,
$X^5$ = Co and/or Sr,
$X^6$ = Si and/or Al,
a = 3 to 4.5,
b = 1 to 1.5,
c = 0.75 to 2.5,
f = 0 to 0.5,
g = 0 to 8, and
n = a number which is determined by the valency and frequency of the elements in II other than oxygen.

11. The process according to any of claims 1 to 10, wherein the thermal treatment of the geometric shaped precursor bodies comprises a calcination at 200 to 600°C.

12. The process according to claim 11, wherein the calcination extends over a period of 0.5 h to 24 h.

13. The process according to any of claims 1 to 12, wherein the catalytically active composition is converted to a finely divided form and the finely divided form is applied as a shell of the catalytically active composition to the outer surface of a geometric shaped support body.

14. The process according to claim 13, wherein the thickness of the shell of catalytically active composition applied is 10 to 1000 $\mu$m.

**Revendications**

1. Procédé de fabrication d'une masse catalytiquement active, qui est un mélange d'un oxyde de plusieurs éléments contenant les éléments Mo et V et d'au moins un oxyde de molybdène, **caractérisé en ce que** le rapport molaire Mo/V, des quantités molaires de Mo et de V contenues dans l'oxyde de plusieurs éléments, est de 15:1 à 1:1 et en tant que mesures de procédé

   - avec des sources des constituants élémentaires de l'oxyde de plusieurs éléments, une solution aqueuse ou une suspension aqueuse est générée, à condition que chacune des sources passe par l'état d'une solution aqueuse lors de la génération de la suspension aqueuse,
   - une poudre pulvérisée P est générée par séchage par pulvérisation de la solution aqueuse ou de la suspension aqueuse,
   - avec la poudre pulvérisée P avec ajout d'au moins un oxyde en poudre S de molybdène, ainsi qu'éventuellement avec ajout d'un ou de plusieurs adjuvants de façonnage et après mélange uniforme des constituants mentionnés, des corps moulés précurseurs géométriques sont formés à partir du mélange alors résultant, ledit au moins un oxyde en poudre S de molybdène étant le dioxyde de molybdène, le trioxyde de molybdène ou un mélange de dioxyde de molybdène et de trioxyde de molybdène, et
   - les corps moulés précurseurs géométriques sont traités thermiquement avec formation de la masse catalytiquement active.

2. Procédé selon la revendication 1, **caractérisé en ce que** la surface spécifique $O_M$ dudit au moins un oxyde en poudre S de molybdène est $\leq$ 20 m²/g.

3. Procédé selon l'une quelconque des revendications 1 ou 2, **caractérisé en ce que** la surface spécifique $O_M$ dudit au moins un oxyde en poudre S de molybdène est $\geq$ 0,01 m²/g.

4. Procédé selon l'une quelconque des revendications 1 à 3, **caractérisé en ce que** le diamètre de particule $d_{90}$ selon ISO 13320 dudit au moins un oxyde en poudre S de molybdène est $\leq$ 20 $\mu$m.

5. Procédé selon l'une quelconque des revendications 1 à 4, **caractérisé en ce que** le diamètre de particule $d_{10}$ selon ISO 13320 dudit au moins un oxyde en poudre S de molybdène est $\geq$ 50 nm.

**6.** Procédé selon l'une quelconque des revendications 1 à 5, **caractérisé en ce que** l'ajout dudit au moins un oxyde en poudre S de molybdène a lieu, par rapport au poids de la masse catalytiquement active fabriquée lors du procédé, en une quantité de > 0 et ≤ 50 % en poids.

**7.** Procédé selon l'une quelconque des revendications 1 à 6, **caractérisé en ce que** la proportion molaire de l'élément Mo par rapport à la quantité totale des éléments différents de l'oxygène de l'oxyde de plusieurs éléments contenant les éléments Mo et V est de 5 à 95 % en moles.

**8.** Procédé selon l'une quelconque des revendications 1 à 7, **caractérisé en ce que** l'oxyde de plusieurs éléments contient en outre encore du Cu.

**9.** Procédé selon l'une quelconque des revendications 1 à 6, **caractérisé en ce que** la stœchiométrie de l'oxyde de plusieurs éléments satisfait la stœchiométrie générale I suivante,

$$Mo_{12}V_aX^1_bX^2_cX^3_dX^4_e,X^5_fX^6_gO_n \qquad (I),$$

dans laquelle les variables ont la signification suivante :

$X^1$ = W, Nb, Ta, Cr et/ou Ce,
$X^2$ = Cu, Ni, Co, Fe, Mn et/ou Zn,
$X^3$ = Sb et/ou Bi,
$X^4$ = un ou plusieurs métaux alcalins (Li, Na, K, Rb, Cs) et/ou H,
$X^5$ = un ou plusieurs métaux alcalino-terreux (Mg, Ca, Sr, Ba),
$X^6$ = Si, Al, Ti et/ou Zr,
a = 1 à 6,
b = 0, 2 à 4,
c = 0 à 18, de préférence 0,5 à 18,
d = 0 à 40,
e = 0 à 2,
f = 0 à 4,
g = 0 à 40, et
n = un nombre, qui est déterminé par la valence et la fréquence des éléments différents de l'oxygène dans I.

**10.** Procédé selon l'une quelconque des revendications 1 à 6, **caractérisé en ce que** la stœchiométrie de l'oxyde de plusieurs éléments satisfait la stœchiométrie générale II suivante,

$$Mo_{12}V_aX^1_bX^2_cX^5_fX^6_gO_n \qquad (II),$$

dans laquelle les variables ont la signification suivante :

$X^1$ = W et/ou Nb,
$X^2$ = Cu et/ou Ni,
$X^5$ = Co et/ou Sr,
$X^6$ = Si et/ou Al,
a = 3 à 4, 5,
b = 1 à 1,5,
c = 0,75 à 2,5,
f = 0 à 0,5,
g = 0 à 8, et
n = un nombre, qui est déterminé par la valence et la fréquence des éléments différents de l'oxygène dans II.

**11.** Procédé selon l'une quelconque des revendications 1 à 10, **caractérisé en ce que** le traitement thermique des corps moulés précurseurs géométriques comprend une calcination à 200 à 600 °C.

**12.** Procédé selon la revendication 11, **caractérisé en ce que** la calcination s'étend sur une durée de 0,5 h à 24 h.

**13.** Procédé selon l'une quelconque des revendications 1 à 12, **caractérisé en ce que** la masse catalytiquement active est transformée en une forme finement divisée et la forme finement divisée est appliquée en tant qu'enveloppe en la masse catalytiquement active sur la surface extérieure d'un corps moulé support géométrique.

14. Procédé selon la revendication 13, **caractérisé en ce que** l'épaisseur de l'enveloppe en masse catalytiquement active appliquée est de 10 à 1 000 μm.

Figur 1a

EP 2 953 720 B1

Figur 1b

Figur 2a

EP 2 953 720 B1

Figur 2b

Figur 3a

EP 2 953 720 B1

Figur 3b

EP 2 953 720 B1

Figur 4b

Figur 5

Figur 6

Figur 7

Figur 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011134932 A1
- DE 102012207811 A1
- WO 2004108267 A1
- WO 2004108284 A1
- EP 714700 A2
- DE 102005010645 A1
- WO 9511081 A1
- DE 10350822 A1
- US 20060205978 A1
- DE 102004025445 A1
- EP 990636 A1
- EP 1106598 A2
- EP 614872 A1
- WO 2008104577 A1
- WO 2008104577 A
- DE 102011084040 A1
- EP 724481 A1
- DE 10201002832 A1
- DE 19927624 A1
- DE 102008054586 A1
- DE 102008040093 A1
- DE 102008040094 A1
- WO 2006094766 A1
- DE 10360058 A1
- DE 2909671 A1
- DE 10051419 A1
- WO 2005120702 A1
- DE 10360057 A1
- WO 2007082827 A1
- WO 2004085365 A2
- WO 2004085367 A1
- WO 2004085368 A2
- WO 2004085369 A1
- WO 2004085370 A1
- WO 2005016861 A1
- WO 2005047226 A1
- WO 2005042459 A1
- DE 10307983 A1
- DE 19948523 A1
- DE 19910508 A1
- EP 700174 A1
- EP 700893 A1
- DE 19910506 A1
- DE 19948241 A1
- DE 2830765 A1
- DE 2513405 A1
- US 3147084 A
- DE 2201428 A1
- EP 383224 A2
- JP 2007260588 A
- JP 58096041 A
- DE 102004017150 A1
- DE 19952964 A1
- DE 10361456 A1
- EP B468290 A1
- DE A10350822 A1
- DE 102007019597 A1
- DE 602004924 T2
- WO 2006114428 A1
- WO 2011134932 A
- US 61761812 B

**In der Beschreibung aufgeführte Nicht-Patentliteratur**

- Synthese und strukturelle Untersuchungen von Molybdän-, Vanadium- und Wolframoxiden als Referenzverbindungen für die heterogene Katalyse. **DR. ANDREAS BLUME.** Dissertation. Fakultät II Mathematik und Naturwissenschaften der Technischen Universität Berlin, 2004
- *Surface Science,* 1983, vol. 292, 261-6
- *J. Solid State Chem.,* 1966, vol. 124, 104
- *J. Am. Chem. Soc.,* 1938, vol. 60 (2), 309-319
- Database. 532036
- Eigenschaften und Einsatzmöglichkeiten von Aerogelfenstern im Vergleich mit konventionellen sowie evakuierten Fenstern. **GEORGES REBER.** Inauguraldissertation. Philosophischnaturwissenschaftlichen Fakultät der Universität Basel, 1991